# EUROPEAN PATENT APPLICATION

(11) **EP 3 295 959 A1**
(43) Date of publication of application: **21.03.2018**
(21) Application number: 16792227.7
(22) Date of filing: 14.06.2016
(51) Int. Cl.: A61K 48/00, A61P 9/04

(54) **CMLCK GENE INTRODUCTION**

(30) Priority: 08.05.2015 CN 201502324156
(71) Applicant: Zensun (Shanghai) Science & Technology, Co., Ltd., Zhangjiang Hi-Tech Park Pudong Shanghai 201203 (CN)
(72) Inventor: ZHOU, Mingdong, Bexley, New South Wales 2207 (AU)
(74) Representative: Jones Day
(86) International application number: PCT/CN2016/085699
(87) International publication number: WO 2016/180377

(57) **Abstract**

The invention relates to compositions and methods for cMLCK gene transfer. In some aspects, the invention provides compositions and methods for treating advanced heart failure in a subject by cMLCK gene transfer, in particular, by administering to a subject an effective amount of a polynucleotide vector that encodes a cMLCK protein, expressing the cMLCK protein from the polynucleotide vector in the heart cells of the subject, and improving ventricular function of the heart. In other aspects, the invention provides compositions and methods for expressing a cMLCK protein in a subject, in particular, by administering to a subject a polynucleotide vector that encodes the cMLCK protein and expressing the cMLCK protein from the polynucleotide vector in the heart cells of the subject, wherein the subject has advanced heart failure.

SEQUENCE LISTING

<110>  Zensun (Shanghai) Science and Technology Ltd.

<120>  cMLCK »ùÒòµ¼Èë

<130>  11748-0056-888

<140>  to be assigned
<141>  to be filed

<160>  6

<170>  PatentIn version 3.5

<210>  1
<211>  819
<212>  PRT
<213>  Homo sapiens

<400>  1

Met Ser Gly Thr Ser Lys Glu Ser Leu Gly His Gly Gly Leu Pro Gly
1               5                   10                  15

Leu Gly Lys Thr Cys Leu Thr Thr Met Asp Thr Lys Leu Asn Met Leu
            20                  25                  30

Asn Glu Lys Val Asp Gln Leu Leu His Phe Gln Glu Asp Val Thr Glu
        35                  40                  45

Lys Leu Gln Ser Met Cys Arg Asp Met Gly His Leu Glu Arg Gly Leu
    50                  55                  60

His Arg Leu Glu Ala Ser Arg Ala Pro Gly Pro Gly Gly Ala Asp Gly
65                  70                  75                  80

Val Pro His Ile Asp Thr Gln Ala Gly Trp Pro Glu Val Leu Glu Leu
                85                  90                  95

Val Arg Ala Met Gln Gln Asp Ala Ala Gln His Gly Ala Arg Leu Glu
            100                 105                 110

Ala Leu Phe Arg Met Val Ala Ala Val Asp Arg Ala Ile Ala Leu Val
        115                 120                 125

Gly Ala Thr Phe Gln Lys Ser Lys Val Ala Asp Phe Leu Met Gln Gly
    130                 135                 140

Arg Val Pro Trp Arg Arg Gly Ser Pro Gly Asp Ser Pro Glu Glu Asn
145                 150                 155                 160

Lys Glu Arg Val Glu Glu Glu Gly Gly Lys Pro Lys His Val Leu Ser

                165                 170                 175

Thr Ser Gly Val Gln Ser Asp Ala Arg Glu Pro Gly Glu Glu Ser Gln
            180                 185                 190

Lys Ala Asp Val Leu Glu Gly Thr Ala Glu Arg Leu Pro Pro Ile Arg
        195                 200                 205

Ala Ser Gly Leu Gly Ala Asp Pro Ala Gln Ala Val Val Ser Pro Gly
    210                 215                 220

Gln Gly Asp Gly Val Pro Gly Pro Ala Gln Ala Phe Pro Gly His Leu
225                 230                 235                 240

Pro Leu Pro Thr Lys Val Glu Ala Lys Ala Pro Glu Thr Pro Ser Glu
                245                 250                 255

Asn Leu Arg Thr Gly Leu Glu Leu Ala Pro Ala Pro Gly Arg Val Asn
            260                 265                 270

Val Val Ser Pro Ser Leu Glu Val Ala Pro Gly Ala Gly Gln Gly Ala
        275                 280                 285

Ser Ser Ser Arg Pro Asp Pro Glu Pro Leu Glu Glu Gly Thr Arg Leu
    290                 295                 300

Thr Pro Gly Pro Gly Pro Gln Cys Pro Gly Pro Pro Gly Leu Pro Ala
305                 310                 315                 320

Gln Ala Arg Ala Thr His Ser Gly Gly Glu Thr Pro Pro Arg Ile Ser
                325                 330                 335

Ile His Ile Gln Glu Met Asp Thr Pro Gly Glu Met Leu Met Thr Gly
            340                 345                 350

Arg Gly Ser Leu Gly Pro Thr Leu Thr Thr Glu Ala Pro Ala Ala Ala
        355                 360                 365

Gln Pro Gly Lys Gln Gly Pro Pro Gly Thr Gly Arg Cys Leu Gln Ala
    370                 375                 380

Pro Gly Thr Glu Pro Gly Glu Gln Thr Pro Glu Gly Ala Arg Glu Leu
385                 390                 395                 400

Ser Pro Leu Gln Glu Ser Ser Ser Pro Gly Gly Val Lys Ala Glu Glu
                405                 410                 415

Glu Gln Arg Ala Gly Ala Glu Pro Gly Thr Arg Pro Ser Leu Ala Arg
            420                 425                 430

Ser Asp Asp Asn Asp His Glu Val Gly Ala Leu Gly Leu Gln Gln Gly
        435                 440                 445

Lys Ser Pro Gly Ala Gly Asn Pro Glu Pro Glu Gln Asp Cys Ala Ala
    450                 455                 460

Arg Ala Pro Val Arg Ala Glu Ala Val Arg Arg Met Pro Pro Gly Ala
465                 470                 475                 480

Glu Ala Gly Ser Val Val Leu Asp Asp Ser Pro Ala Pro Pro Ala Pro
                485                 490                 495

Phe Glu His Arg Val Val Ser Val Lys Glu Thr Ser Ile Ser Ala Gly
            500                 505                 510

Tyr Glu Val Cys Gln His Glu Val Leu Gly Gly Gly Arg Phe Gly Gln
        515                 520                 525

Val His Arg Cys Thr Glu Lys Ser Thr Gly Leu Pro Leu Ala Ala Lys
    530                 535                 540

Ile Ile Lys Val Lys Ser Ala Lys Asp Arg Glu Asp Val Lys Asn Glu
545                 550                 555                 560

Ile Asn Ile Met Asn Gln Leu Ser His Val Asn Leu Ile Gln Leu Tyr
                565                 570                 575

Asp Ala Phe Glu Ser Lys His Ser Cys Thr Leu Val Met Glu Tyr Val
            580                 585                 590

Asp Gly Gly Glu Leu Phe Asp Arg Ile Thr Asp Glu Lys Tyr His Leu
        595                 600                 605

Thr Glu Leu Asp Val Val Leu Phe Thr Arg Gln Ile Cys Glu Gly Val
    610                 615                 620

His Tyr Leu His Gln His Tyr Ile Leu His Leu Asp Leu Lys Pro Glu
625                 630                 635                 640

Asn Ile Leu Cys Val Asn Gln Thr Gly His Gln Ile Lys Ile Ile Asp
                645                 650                 655

Phe Gly Leu Ala Arg Arg Tyr Lys Pro Arg Glu Lys Leu Lys Val Asn
            660                 665                 670

Phe Gly Thr Pro Glu Phe Leu Ala Pro Glu Val Val Asn Tyr Glu Phe
        675                 680                 685

Val Ser Phe Pro Thr Asp Met Trp Ser Val Gly Val Ile Thr Tyr Met
    690                 695                 700

Leu Leu Ser Gly Leu Ser Pro Phe Leu Gly Glu Thr Asp Ala Glu Thr
705                 710                 715                 720

Met Asn Phe Ile Val Asn Cys Ser Trp Asp Phe Asp Ala Asp Thr Phe
                725                 730                 735

Glu Gly Leu Ser Glu Glu Ala Lys Asp Phe Val Ser Arg Leu Leu Val
            740                 745                 750

Lys Glu Lys Ser Cys Arg Met Ser Ala Thr Gln Cys Leu Lys His Glu
        755                 760                 765

Trp Leu Asn Asn Leu Pro Ala Lys Ala Ser Arg Ser Lys Thr Arg Leu
    770                 775                 780

Lys Ser Gln Leu Leu Leu Gln Lys Tyr Ile Ala Gln Arg Lys Trp Lys
785                 790                 795                 800

Lys His Phe Tyr Val Val Thr Ala Ala Asn Arg Leu Arg Lys Phe Pro
                805                 810                 815

Thr Ser Pro

<210>  2
<211>  795
<212>  PRT
<213>  Homo sapiens

<400>  2

Met Asp Thr Lys Leu Asn Met Leu Asn Glu Lys Val Asp Gln Leu Leu
1               5                   10                  15

His Phe Gln Glu Asp Val Thr Glu Lys Leu Gln Ser Met Cys Arg Asp
            20                  25                  30

Met Gly His Leu Glu Arg Gly Leu His Arg Leu Glu Ala Ser Arg Ala
        35                  40                  45

Pro Gly Pro Gly Gly Ala Asp Gly Val Pro His Ile Asp Thr Gln Ala
    50                  55                  60

Gly Trp Pro Glu Val Leu Glu Leu Val Arg Ala Met Gln Gln Asp Ala
65                  70                  75                  80

Ala Gln His Gly Ala Arg Leu Glu Ala Leu Phe Arg Met Val Ala Ala
                85                  90                  95

Val Asp Arg Ala Ile Ala Leu Val Gly Ala Thr Phe Gln Lys Ser Lys
            100                 105                 110

Val Ala Asp Phe Leu Met Gln Gly Arg Val Pro Trp Arg Arg Gly Ser
        115                 120                 125

Pro Gly Asp Ser Pro Glu Glu Asn Lys Glu Arg Val Glu Glu Glu Gly
    130                 135                 140

Gly Lys Pro Lys His Val Leu Ser Thr Ser Gly Leu Gln Ser Asp Ala
145                 150                 155                 160

Arg Glu Pro Gly Glu Glu Ser Gln Lys Ala Asp Val Leu Glu Arg Thr
                165                 170                 175

Ala Glu Arg Leu Pro Pro Ile Arg Ala Ser Gly Leu Gly Ala Asp Pro
            180                 185                 190

Ala Gln Ala Val Val Ser Pro Gly Gln Gly Asp Gly Val Pro Gly Pro
        195                 200                 205

Ala Gln Ala Phe Pro Gly His Leu Pro Leu Pro Thr Lys Val Glu Ala
    210                 215                 220

Lys Ala Pro Glu Thr Pro Ser Glu Asn Leu Arg Thr Gly Leu Glu Leu
225                 230                 235                 240

Ala Pro Ala Pro Gly Arg Val Asn Val Val Ser Pro Ser Leu Glu Val
                245                 250                 255

Ala Pro Gly Ala Gly Gln Gly Ala Ser Ser Ser Arg Pro Asp Pro Glu
            260                 265                 270

Pro Leu Glu Glu Gly Thr Arg Leu Thr Pro Gly Pro Gly Pro Gln Cys
        275                 280                 285

Pro Gly Pro Pro Gly Leu Pro Ala Gln Ala Arg Ala Thr His Ser Gly
    290                 295                 300

Gly Glu Thr Pro Pro Arg Ile Ser Ile His Ile Gln Glu Met Asp Thr
305                 310                 315                 320

Pro Gly Glu Met Leu Met Thr Gly Arg Gly Ser Leu Gly Pro Thr Leu
                325                 330                 335

Thr Thr Glu Ala Pro Ala Ala Ala Gln Pro Gly Lys Gln Gly Pro Pro
            340                 345                 350

Gly Thr Gly Arg Cys Leu Gln Ala Pro Gly Thr Glu Pro Gly Glu Gln
        355                 360                 365

Thr Pro Glu Gly Ala Arg Glu Leu Ser Pro Leu Gln Glu Ser Ser Ser
    370                 375                 380

Pro Gly Gly Val Lys Ala Glu Glu Glu Gln Arg Ala Gly Ala Glu Pro
385                 390                 395                 400

Gly Thr Arg Pro Ser Leu Ala Arg Ser Asp Asp Asn Asp His Glu Val
                405                 410                 415

Gly Ala Leu Gly Leu Gln Gln Gly Lys Ser Pro Gly Ala Gly Asn Pro
            420                 425                 430

Glu Pro Glu Gln Asp Cys Ala Ala Arg Ala Pro Val Arg Ala Glu Ala
        435                 440                 445

Val Arg Arg Met Pro Pro Gly Ala Glu Ala Gly Ser Val Val Leu Asp
    450                 455                 460

Asp Ser Pro Ala Pro Pro Ala Pro Phe Glu His Arg Val Val Ser Val
465                 470                 475                 480

Lys Glu Thr Ser Ile Ser Ala Gly Tyr Glu Val Cys Gln His Glu Val
                485                 490                 495

Leu Gly Gly Gly Arg Phe Gly Gln Val His Arg Cys Thr Glu Lys Ser
            500                 505                 510

Thr Gly Leu Pro Leu Ala Ala Lys Ile Ile Lys Val Lys Ser Ala Lys
        515                 520                 525

Asp Arg Glu Asp Val Lys Asn Glu Ile Asn Ile Met Asn Gln Leu Ser
    530                 535                 540

His Val Asn Leu Ile Gln Leu Tyr Asp Ala Phe Glu Ser Lys His Ser
545                 550                 555                 560

Cys Thr Leu Val Met Glu Tyr Val Asp Gly Gly Glu Leu Phe Asp Arg

                565                 570                 575

Ile Thr Asp Glu Lys Tyr His Leu Thr Glu Leu Asp Val Val Leu Phe
            580                 585                 590

Thr Arg Gln Ile Cys Glu Gly Val His Tyr Leu His Gln His Tyr Ile
        595                 600                 605

Leu His Leu Asp Leu Lys Pro Glu Asn Ile Leu Cys Val Asn Gln Thr
    610                 615                 620

Gly His Gln Ile Lys Ile Ile Asp Phe Gly Leu Ala Arg Arg Tyr Lys
625                 630                 635                 640

Pro Arg Glu Lys Leu Lys Val Asn Phe Gly Thr Pro Glu Phe Leu Ala
                645                 650                 655

Pro Glu Val Val Asn Tyr Glu Phe Val Ser Phe Pro Thr Asp Met Trp
            660                 665                 670

Ser Val Gly Val Ile Thr Tyr Met Leu Leu Ser Gly Leu Ser Pro Phe
        675                 680                 685

Leu Gly Glu Thr Asp Ala Glu Thr Met Asn Phe Ile Val Asn Cys Ser
    690                 695                 700

Trp Asp Phe Asp Ala Asp Thr Phe Glu Gly Leu Ser Glu Glu Ala Lys
705                 710                 715                 720

Asp Phe Val Ser Arg Leu Leu Val Lys Glu Lys Ser Cys Arg Met Ser
                725                 730                 735

Ala Thr Gln Cys Leu Lys His Glu Trp Leu Asn Asn Leu Pro Ala Lys
            740                 745                 750

Ala Ser Arg Ser Lys Thr Arg Leu Lys Ser Gln Leu Leu Leu Gln Lys
        755                 760                 765

Tyr Ile Ala Gln Arg Lys Trp Lys Lys His Phe Tyr Val Val Thr Ala
    770                 775                 780

Ala Asn Arg Leu Arg Lys Phe Pro Thr Ser Pro
785                 790                 795

<210> 3
<211> 786
<212> PRT
<213> Mus musculus

<400> 3

Met Ser Gly Val Ser Glu Glu Asp Pro Glu Gly Leu Gly Pro Gln Gly
1               5                   10                  15

Leu Pro Ala Leu Gly Gly Ala Cys Leu Val Thr Val Asp Lys Lys Leu
            20                  25                  30

Asn Val Leu Thr Glu Lys Val Asp Arg Leu Leu His Phe Gln Glu Asp
        35                  40                  45

Val Thr Glu Lys Leu Gln Cys Val Cys Gln Gly Met Asp His Leu Glu
    50                  55                  60

Gln Gly Leu His Arg Leu Glu Ala Ser Gln Glu Leu Gly Leu Ala Gly
65                  70                  75                  80

Pro Gly Ser Thr Ser Pro Ala Ala Ala Gln Ala Ala Trp Pro Glu Val
                85                  90                  95

Leu Glu Leu Val Arg Ala Val Arg Gln Glu Gly Ala Gln His Gly Ala
            100                 105                 110

Arg Leu Glu Ala Leu Phe Lys Met Val Val Ala Val Asp Arg Ala Ile
        115                 120                 125

Thr Leu Val Gly Ser Thr Ile Gln Asn Ser Lys Val Asp Asp Phe Ile
    130                 135                 140

Leu Gln Gly Thr Val Pro Trp Arg Lys Gly Ser Leu Ala Asp Gly Pro
145                 150                 155                 160

Glu Glu Asn Lys Glu Gln Ala Glu Val Ala Gly Val Lys Pro Lys His
                165                 170                 175

Val Leu Asn Thr Gly Ser Val Gln Ala Ala Thr Ser Arg Ala Leu Trp
            180                 185                 190

Glu Glu Ser Gln Lys Gln Asp Thr Pro Val Gly Thr Val Glu Gly Leu
        195                 200                 205

Pro Leu Ile Ile Asp Thr Ser Leu Lys Gly Ala Asp Leu Thr Gln Ala
    210                 215                 220

Gly Ala Ser Leu Arg Gln Gly Val Glu Ala Leu Asp Pro Gly Gln Glu
225                 230                 235                 240

Pro Pro Pro Thr Glu Ala Glu Ser Arg Leu Pro Ala Leu Ala Ser Glu
                245                 250                 255

Asp Thr Gly Thr Thr Leu Glu Leu Ser Val Ala Ile Asp Arg Ile Ser
            260                 265                 270

Glu Val Leu Thr Ser Leu Arg Met Ser Gln Ser Ala Gly Glu Gly Thr
        275                 280                 285

Ser Ser Ser Lys Pro Asp Cys Ser Glu Pro Gly Pro Gln Pro Leu Gly
    290                 295                 300

Pro Leu Thr Thr Asp Ser Asp Ile His Ser Asp Glu Gly Leu Pro Arg
305                 310                 315                 320

Ile Ser Val Arg Met Arg Glu Met Thr Thr Pro Glu Glu Leu Phe Glu
                325                 330                 335

Thr Gln Gly Gly Ser Pro Ile Gly Ser Ala Glu Ala Pro Gly Pro Gly
            340                 345                 350

Thr Val Leu Glu Asp Gln Ile Pro Lys Gly Ala Arg Pro Phe Pro Pro
        355                 360                 365

Leu Pro Lys Arg Ser Cys Asn Asn Gly Gly Ala Ser Ala Glu Glu Ala
    370                 375                 380

Thr Gly Pro Gly Ala Glu Pro Ile Arg Gly Pro Ser Leu Val Thr Arg
385                 390                 395                 400

Asp Trp Arg Asp Glu Pro Val Gly Thr Thr Asp Leu Gln Gln Gly Arg
                405                 410                 415

Asp Pro Gly Ala Val Ser Pro Glu Pro Gly Lys Asp His Ala Ala Gln
            420                 425                 430

Gly Pro Gly Arg Thr Glu Ala Gly Arg Arg Val Ser Ser Ala Ala Glu
        435                 440                 445

Ala Ala Ile Val Val Leu Gly Asp Ser Ala Ala Pro Pro Ala Pro Phe
    450                 455                 460

Glu His Arg Val Val Ser Ile Lys Asp Thr Leu Ile Ser Thr Ser Tyr
465                 470                 475                 480

Thr Val Ser Gln His Glu Val Leu Gly Gly Gly Arg Phe Gly Gln Val
                485                 490                 495

His Arg Cys Thr Glu Arg Ser Thr Gly Leu Ala Leu Ala Ala Lys Ile
                500                 505                 510

Ile Lys Val Lys Asn Ile Lys Asp Arg Glu Asp Val Lys Asn Glu Ile
            515                 520                 525

Asn Ile Met Asn Gln Leu Ser His Val Asn Leu Ile Gln Leu Tyr Asp
        530                 535                 540

Ala Phe Glu Ser Lys Asn Ser Phe Thr Leu Ile Met Glu Tyr Val Asp
545                 550                 555                 560

Gly Gly Glu Leu Phe Asp Arg Ile Thr Asp Glu Lys Tyr His Leu Thr
                565                 570                 575

Glu Leu Asp Val Val Leu Phe Thr Arg Gln Ile Cys Glu Gly Val His
            580                 585                 590

Tyr Leu His Gln His Tyr Ile Leu His Leu Asp Leu Lys Pro Glu Asn
        595                 600                 605

Ile Leu Cys Val Ser Gln Thr Gly His Gln Ile Lys Ile Ile Asp Phe
    610                 615                 620

Gly Leu Ala Arg Arg Tyr Lys Pro Arg Glu Lys Leu Lys Val Asn Phe
625                 630                 635                 640

Gly Thr Pro Glu Phe Leu Ala Pro Glu Val Val Asn Tyr Glu Phe Val
                645                 650                 655

Ser Phe Pro Thr Asp Met Trp Ser Val Gly Val Ile Thr Tyr Met Leu
            660                 665                 670

Leu Ser Gly Leu Ser Pro Phe Leu Gly Glu Thr Asp Ala Glu Thr Met
        675                 680                 685

Asn Phe Ile Val Asn Cys Ser Trp Asp Phe Asp Ala Asp Thr Phe Lys
    690                 695                 700

Gly Leu Ser Glu Glu Ala Lys Asp Phe Val Ser Arg Leu Leu Val Lys
705                 710                 715                 720

Glu Lys Ser Cys Arg Met Ser Ala Thr Gln Cys Leu Lys His Glu Trp
                725                 730                 735

Leu Asn His Leu Ile Ala Lys Ala Ser Gly Ser Asn Val Arg Leu Arg
            740                 745                 750

Ser Gln Leu Leu Leu Gln Lys Tyr Met Ala Gln Arg Lys Trp Lys Lys
        755                 760                 765

His Phe His Val Val Thr Ala Val Asn Arg Leu Arg Lys Phe Pro Thr
    770                 775                 780

Cys Pro
785

<210>  4
<211>  2457
<212>  DNA
<213>  Homo sapiens

<400>  4
atgtcaggaa cctccaagga gagtctgggg catggggggc tgccagggtt gggcaagacc      60

tgcttaacaa ccatggacac aaagctgaac atgctgaacg agaaggtgga ccagctcctg     120

cacttccaag aagatgtcac agagaagttg cagagcatgt gccgagacat gggccacctg     180

gagcggggcc tgcacaggct ggaggcctcc cgggcaccgg gcccgggcgg ggctgatggg     240

gttccccaca ttgacaccca ggctgggtgg cccgaggtcc tggagctggt gagggccatg     300

cagcaggatg cggcccagca cggtgccagg ctggaggccc tcttcaggat ggtggctgcg     360

gtggacaggg ccatcgcttt ggtgggggcc acgttccaga aatcaaaggt ggcggatttc     420

ctcatgcagg ggcgtgtgcc ctggaggaga ggcagcccag gtgacagccc tgaggagaat     480

aaagagcgag tggaagaaga gggaggaaaa ccaaagcatg tgctgagcac cagtggggtg     540

cagtctgatg ccagggagcc tggggaagag agccagaagg cggacgtgct ggaggggaca     600

gcggagaggc tgccccccat cagagcgtca gggctgggag ctgaccccgc ccaggcagtg     660

gtctcaccgg gccagggaga tggtgttcct ggcccagccc aggcattccc tggccacctg     720

cccctgccca caaaggtgga agccaaggct cctgagacac ccagcgagaa cctcaggact     780

ggcctggaat tggctccagc acccggcagg gtcaatgtgg tctccccgag cctggaggtt     840

gcaccaggtg caggacaagg agcatcgtcc agcaggcctg accctgagcc cttagaggaa     900

ggcacgaggc tgactccagg gcctggccct cagtgcccag ggcctccagg gctgccagcc     960

caggccaggg caacccacag tggtggagaa acacctccaa ggatctccat ccacatacaa    1020

gagatggata ctcctgggga gatgctgatg acaggcaggg gcagccttgg acccaccctc    1080

accacagagg ctccagcagc tgcccagcca ggcaagcagg gcccacctgg gaccgggcgc    1140

tgcctccaag cccctgggac tgagcccgga gaacagaccc ctgaaggagc cagagagctc    1200

tccccgctgc aggagagcag cagccccggg ggagtgaagg cagaggagga gcaaagggct    1260

ggggccgagc ctggcacgag accaagcttg gccaggagtg acgacaatga ccacgaggtt    1320

ggggccctgg gcctgcagca gggcaaaagc ccaggggcgg gaaaccctga gcctgagcag 1380

gactgtgcag ccagggctcc ggtgagagct gaagcagtaa ggaggatgcc cccaggcgcc 1440

gaggctggca gcgtggttct ggatgacagt ccggccccac cagctccttt tgaacaccgg 1500

gtagtgagcg tcaaggagac ctccatctct gcgggttacg aggtgtgcca gcacgaagtc 1560

ttgggagggg gtcggtttgg ccaggtccac aggtgcacag agaagtccac aggcctccca 1620

ctggctgcca agatcatcaa agtgaagagc gccaaggacc gggaggacgt gaagaacgag 1680

atcaacatca tgaaccagct cagccacgtg aacctgatcc agctctatga cgccttcgag 1740

agcaagcaca gctgcaccct tgtcatggag tacgtggacg ggggtgagct cttcgaccgg 1800

atcacagatg agaagtacca cctgactgag ctggatgtgg tcctgttcac caggcagatc 1860

tgtgagggtg tgcattacct gcaccagcac tacatcctgc acctggacct caagccggag 1920

aacatattgt gcgtcaatca gacaggacat caaattaaga tcattgactt tgggctggcc 1980

agaaggtaca agcctcgaga gaagctgaag gtgaacttcg gcactcctga gttcctggcc 2040

ccagaagtcg tcaattatga gtttgtctca ttccccacag acatgtggag tgtgggagtc 2100

atcacctaca tgctactcag tggcttgtcc ccatttctag gggaaacaga tgcagagacc 2160

atgaatttca ttgtaaactg tagctgggat tttgatgctg acacctttga agggctctcg 2220

gaggaggcca aggactttgt ttcccggttg ctggtcaaag agaagagctg cagaatgagt 2280

gccacacagt gcctgaaaca cgagtggctg aataatttgc ctgccaaagc ttcaagatcc 2340

aaaactcgtc tcaaatccca actactgctg cagaaataca tagctcaaag aaaatggaag 2400

aaacatttct atgtggtgac tgctgccaac aggttaagga aatttccaac ttctccc 2457

<210> 5
<211> 2385
<212> DNA
<213> Homo sapiens

<400> 5
atggacacaa agctgaacat gctgaacgag aaggtggacc agctcctgca cttccaagaa 60

gatgtcacag agaagttgca gagcatgtgc cgagacatgg gccacctgga gcggggcctg 120

cacaggctgg aggcctcccg ggcaccgggc ccgggcgggg ctgatggggt tccccacatt 180

gacacccagg ctgggtggcc cgaggtcctg gagctggtga gggccatgca gcaggatgcg 240

gcccagcacg gtgccaggct ggaggccctc ttcaggatgg tggctgcggt ggacagggcc 300

atcgctttgg tgggggccac gttccagaaa tcaaaggtgg cggatttcct catgcagggg 360

cgtgtgccct ggaggagagg cagcccaggt gacagccctg aggagaataa agagcgagtg 420

gaagaagagg gaggaaaacc aaagcatgtg ctgagcacca gtggggtgca gtctgatgcc 480

agggagcctg gggaagagag ccagaaggcg gacgtgctgg aggggacagc ggagaggctg 540

ccccccatca gagcgtcagg gctgggagct gaccccgccc aggcagtggt ctcaccgggc 600
cagggagatg gtgttcctgg cccagcccag gcattccctg gccacctgcc cctgcccaca 660
aaggtggaag ccaaggctcc tgagacaccc agcgagaacc tcaggactgg cctggaattg 720
gctccagcac ccggcagggt caatgtggtc tccccgagcc tggaggttgc accaggtgca 780
ggacaaggag catcgtccag caggcctgac cctgagccct tagaggaagg cacgaggctg 840
actccagggc ctggccctca gtgcccaggg cctccagggc tgccagccca ggccagggca 900
acccacagtg gtggagaaac acctccaagg atctccatcc acatacaaga gatggatact 960
cctggggaga tgctgatgac aggcaggggc agccttggac ccaccctcac cacagaggct 1020
ccagcagctg cccagccagg caagcagggc ccacctggga ccgggcgctg cctccaagcc 1080
cctgggactg agcccggaga acagacccct gaaggagcca gagagctctc cccgctgcag 1140
gagagcagca gccccggggg agtgaaggca gaggaggagc aaagggctgg ggccgagcct 1200
ggcacgagac caagcttggc caggagtgac gacaatgacc acgaggttgg ggccctgggc 1260
ctgcagcagg gcaaaagccc aggggcggga aaccctgagc ctgagcagga ctgtgcagcc 1320
agggctccgg tgagagctga agcagtaagg aggatgcccc caggcgccga ggctggcagc 1380
gtggttctgg atgacagtcc ggccccacca gctccttttg aacaccgggt agtgagcgtc 1440
aaggagacct ccatctctgc gggttacgag gtgtgccagc acgaagtctt gggagggggt 1500
cggtttggcc aggtccacag gtgcacagag aagtccacag gcctcccact ggctgccaag 1560
atcatcaaag tgaagagcgc caaggaccgg gaggacgtga agaacgagat caacatcatg 1620
aaccagctca gccacgtgaa cctgatccag ctctatgacg ccttcgagag caagcacagc 1680
tgcacccttg tcatggagta cgtggacggg ggtgagctct tcgaccggat cacagatgag 1740
aagtaccacc tgactgagct ggatgtggtc ctgttcacca ggcagatctg tgagggtgtg 1800
cattacctgc accagcacta catcctgcac ctggacctca agccggagaa catattgtgc 1860
gtcaatcaga caggacatca aattaagatc attgactttg ggctggccag aaggtacaag 1920
cctcgagaga agctgaaggt gaacttcggc actcctgagt tcctggcccc agaagtcgtc 1980
aattatgagt ttgtctcatt ccccacagac atgtggagtg tgggagtcat cacctacatg 2040
ctactcagtg gcttgtcccc atttctaggg gaaacagatg cagagaccat gaatttcatt 2100
gtaaactgta gctgggattt tgatgctgac acctttgaag ggctctcgga ggaggccaag 2160
gactttgttt cccggttgct ggtcaaagag aagagctgca gaatgagtgc cacacagtgc 2220
ctgaaacacg agtggctgaa taatttgcct gccaaagctt caagatccaa aactcgtctc 2280
aaatcccaac tactgctgca gaaatacata gctcaaagaa aatggaagaa acatttctat 2340
gtggtgactg ctgccaacag gttaaggaaa tttccaactt ctccc 2385

<210>  6
<211>  2358
<212>  DNA
<213>  Mus musculus

<400>  6
atgtcaggag tttcagagga ggatccagag ggtctgggcc cccagggtct gccagcgttg      60

ggcggagcct gcttagtcac cgtggacaaa aaacttaatg tgctgactga gaaggtcgac     120

agactcttgc atttccaaga agatgtcaca gagaagctac agtgtgtgtg ccaaggcatg     180

gatcacctgg aacaaggtct gcatcggctg gaggcctccc aggagttggg tctggcaggg     240

cccggcagca cttccccagc cgctgctcag gccgcatggc ctgaggtcct ggagctggtg     300

agggccgtgc ggcaggaggg tgcccagcac ggtgccaggc tcgaagccct cttcaagatg     360

gtggtggctg tggacagggc tattactttg gtagggtcca caatccagaa ctccaaagtg     420

gatgatttca tcctgcaagg gaccgtgccc tggaggaaag gcagtctggc tgatggccct     480

gaggagaaca aggagcaagc agaagtggct ggagtgaagc caaagcatgt gctgaataca     540

ggaagtgtgc aagctgccac ttctagggcg ctgtgggaag agagccagaa gcaggacaca     600

cccgtgggga cagtggaggg gctgcctctc atcatcgata cgtcactgaa gggagctgac     660

ctcacccagg ctggagcctc actgaggcag ggagttgaag ctcttgaccc aggccaagaa     720

cccccacccg cagaggcaga atccaggctt cctgcactag ccagcgagga cactgggacc     780

accctggaat tgtctgtagc aattgacaga atcagtgagg tcctcactag cctcaggatg     840

tcccaaagtg ctggcgaagg aacctcatcc agcaagcctg actgttcaga gcctggccct     900

cagcccctag ggccactaac tacagacagt gacattcaca gtgatgaagg acttcccagg     960

atctctgtcc gtatgcgaga gatgactact cctgaggagc tgtttgagac ccaaggtggc    1020

agccccattg gctcggcaga agctccaggc cctggaactg tgttagaaga ccagatccct    1080

aaaggagcca gaccatttcc acccctgcca aagaggagct gcaacaatgg tggcgcgagt    1140

gcagaggagg caacagggcc tggggctgag cccatcagag gaccaagctt ggtcacaagg    1200

gactggagag atgaacctgt tgggaccaca gacctgcagc aaggcagaga cccaggagcg    1260

gtgagccctg aacctgggaa ggaccatgca gcccagggcc cagggagaac cgaagctgga    1320

aggagggtgt cttctgctgc agaggctgcc atcgtagttc tagatgacag cgcagcaccc    1380

ccagcccctt ttgaacaccg ggtagtgagc atcaaagaca ccctcatctc gacaagttac    1440

acagtgtccc aacacgaagt cttgggaggg ggccggtttg gccaggtgca caggtgtaca    1500

gagcggtcca caggccttgc actggcagcc aagatcatca aagtgaagaa cataaaggac    1560

cgggaggatg tgaagaatga gatcaacatc atgaaccagc tcagccacgt aaacttgatc    1620

caactttatg atgcttttga gagcaagaac agcttcaccc tgatcatgga gtatgtggat    1680

ggaggtgaac tcttcgaccg gatcacggat gagaagtacc acctgaccga gctggatgtg 1740

gtcttgttca caaggcagat ctgtgagggt gtgcattacc tgcaccagca ctacattctg 1800

cacctggacc tcaagccaga gaacatactg tgtgtcagcc agactgggca tcaaattaag 1860

attattgact ttgggctggc tagaagatat aaacctcggg agaagctaaa ggttaacttt 1920

ggtactccag agttcctggc tccagaagtt gttaactatg agtttgtctc attcccaaca 1980

gacatgtgga gtgtgggagt tatcacctac atgctactca gtggtttgtc cccatttcta 2040

ggggaaacag atgcagagac catgaatttt attgtgaact gcagctggga tttcgatgct 2100

gataccttca aagggctgtc agaggaagcc aaggactttg tttcacggtt gctggtcaaa 2160

gagaagagtt gtaggatgag cgccacacag tgcctgaaac atgagtggtt aaatcacctg 2220

attgccaaag cctcaggctc caacgttcgc ctcagatccc aactactgct gcagaaatat 2280

atggctcagc gtaaatggaa gaaacatttc catgtggtga ctgcagtcaa caggctaaga 2340

aaatttccaa cgtgtccc 2358

## Description

### FIELD OF THE INVENTION

The invention relates to compositions and methods for cMLCK gene transfer. In some aspects, the invention provides compositions and methods for treating advanced heart failure in a subject by cMLCK gene transfer, in particular, by administering to a subject an effective amount of a polynucleotide vector that encodes a cMLCK protein, expressing the cMLCK protein from the polynucleotide vector in the heart cells of the subject, and improving ventricular function of the heart. In other aspects, the invention provides compositions and methods for expressing a cMLCK protein in a subject, in particular, by administering to a subject a polynucleotide vector that encodes the cMLCK protein and expressing the cMLCK protein from the polynucleotide vector in the heart cells of the subject, wherein the subject has advanced heart failure.

### BACKGROUND OF THE INVENTION

Heart failure affects approximately five million Americans, and more than 550,000 new patients are diagnosed with the condition each year. Current drug therapy for heart failure is primarily directed to angiotensin-converting enzyme (ACE) inhibitors, which are vasodilators that cause blood vessels to expand, lowering blood pressure and reducing the heart's workload. While the percent reduction in mortality has been significant, the actual reduction in mortality with ACE inhibitors has averaged only 3%-4%, and there are several potential side effects. Additional limitations are associated with other options for preventing or treating heart failure. For example, heart transplantation is clearly more expensive and invasive than drug treatment, and it is further limited by the availability of donor hearts. Uses of mechanical devices, such as biventricular pacemakers, are similarly invasive and expensive. Thus, there has been a need for new therapies given the deficiencies in current therapies.

One promising new therapy involves administration of neuregulin (hereinafter referred to as "NRG") to a patient suffering from or at risk of developing heart failure. NRGs, a family of EGF-like growth factors, comprises a family of structurally related growth and differentiation factors that include NRG1, NRG2, NRG3 and NRG4 and isoforms thereof, are involved in an array of biological responses: stimulation of breast cancer cell differentiation and secretion of milk proteins; induction of neural crest cell differentiation to Schwann cells; stimulation of skeletal muscle cell synthesis of acetylcholine receptors; and, promotion of myocardial cell survival and DNA synthesis. *In vivo* studies of neuregulin gene-targeted homozygous mouse embryos with severe defects in ventricular trabeculae formation and dorsal root ganglia development indicate that neuregulin is essential for heart and neural development.

NRGs bind to the EGF receptor family, which comprises EGFR, ErbB2, ErbB3 and ErbB4, each of which plays an important role in multiple cellular functions, including cell growth, differentiation, and survival. They are protein tyrosine kinase receptors, consisting of an extracellular ligand-binding domain, transmembrane kinase domain and cytoplasmic tyrosine kinase domain. After NRG binds to the extracellular domain of ErbB3 or ErbB4, it induces a conformational change that leads to heterodimer formation between ErbB3, ErbB4 and ErbB2 or homodimer formation between ErbB4 itself, which results in phosphorylation of the receptor's C-terminal domain inside the cell membrane. The phosphorylated intracellular domain then binds additional signal proteins inside the cell, activating the corresponding downstream AKT or ERK signaling pathway, and inducing a series of cell reactions, such as stimulation or depression of cell proliferation, cell differentiation, cell apoptosis, cell migration or cell adhesion. Among these receptors, mainly ErbB2 and ErbB4 are expressed in the heart.

It has been shown that the EGF-like domains of NRG-1, ranging in size from 50 to 64-amino acids, are sufficient to bind to and activate these receptors. Previous studies have shown that neuregulin-β (NRG-1β) can bind directly to ErbB3 and ErbB4 with high affinity. The orphan receptor, ErbB2, can form heterodimer with ErbB3 and ErbB4 with higher affinity than ErbB3 or ErbB4 homodimers. Research in neural development has indicated that the formation of the sympathetic nervous system requires an intact NRG-1β, ErbB2 and ErbB3 signaling system. Targeted disruption of the NRG-1β or ErbB2 or ErbB4 led to embryonic lethality due to cardiac development defects. Recent studies also highlighted the roles of NRG-1β, ErbB2 and ErbB4 in the cardiovascular development as well as in the maintenance of adult normal heart function. NRG-1β has been shown to enhance sarcomere organization in adult cardiomyocytes. Extended release of NRG via intravenous infusion significantly improves or protects against deterioration in myocardial performance in distinct animal models of heart failure as well as in clinical trials. These results make NRG-1 promising as a lead compound for the treatment of heart failure.

Zensun has reported that neuregulin enhances cardiac myosin light chain kinase (cMLCK) expression and cardiac myosin regulatory light chain (RLC) phosphorylation, which may improve actin-myosin interaction for contraction, and that cMLCK could serve as a potential therapeutic target for heart failure (*see, e.g.,* WO 08/28405). In Gu et al., Cardiovascular Research, 88:334-343 (2010), Zensun has reported that the up-regulation of cMLCK could promote sarcomere reassembly and enhance contractility of the failing heart; however, cMLCK gene therapy was not as effective as neuregulin delivery in rats with myocardial infarction.

However, there is a need for methods for the treatment of heart failure if neuregulin treatment is not effective.

### SUMMARY OF EMBODIMENTS

The present disclosure provides compositions and methods for cMLCK gene transfer. In some aspects, the invention provides methods for treating advanced heart failure in a subject, comprising administering to the subject an effective amount of a polynucleotide vector that encodes a cMLCK protein, expressing the cMLCK protein from the polynucleotide vector in the heart cells of the subject, and improving ventricular function of the heart. In some embodiments, the subject is a human.

In some aspects, the invention provides methods for expressing a cMLCK protein in a subject, comprising administering to the subject a polynucleotide vector that encodes the cMLCK protein and expressing the cMLCK protein from the polynucleotide vector in the heart cells of the subject, wherein the subject has advanced heart failure. In some embodiments, the subject is a human. In some embodiments, the subject has an improvement in ventricular function of the heart.

In some embodiments, the cMLCK-encoding polynucleotide vector is systemically administered to the subject. In other embodiments, the cMLCK-encoding polynucleotide vector is locally administered to the subject.

In some embodiments, the cMLCK-encoding polynucleotide vector is locally administered into the subject's heart via intracoronary infusion. In some embodiments, the cMLCK-encoding polynucleotide vector is locally administered into the subject's LV cavity. In other embodiments, the cMLCK-encoding polynucleotide vector is locally administered into the subject's heart surgically (*i.e.,* by direct introduction of the vector or transfected cells into the subject's heart). In yet other embodiments, the cMLCK-encoding polynucleotide vector is administered to the heart of the subject in a liposome.

The subject in the disclosed methods is assessed for improvements of ventricular function of the heart. In some embodiments, the EF values of the left ventricle of the subject is enhanced by at least 5%. In some embodiments, the EF values of the left ventricle of the subject is enhanced by at least 10%. In some embodiments, the EF values of the left ventricle of the subject is enhanced by at least 20%. In some embodiments, the Left Ventricular End-Diastolic Volume (LVEDV) or Left Ventricular End-Systolic Volume (LVESV) of the subject is reduced by at least 5%. In some embodiments, the LVEDV or LVESV of the subject is reduced by at least 10%. In some embodiments, the LVEDV or LVESV of the subject is reduced by at least 20%.

In some embodiments, the polynucleotide vector encodes a polypeptide comprising an amino acid sequence having at least 70% identity to SEQ ID NO:1, SEQ ID NO:2, or SEQ ID NO:3. In some embodiments, the polynucleotide vector encodes a polypeptide comprising an amino acid sequence having at least 75%, 80%, 85%, 90%, or 95% identity to SEQ ID NO:1, SEQ ID NO:2, or SEQ ID NO:3. In a particular embodiment, the polynucleotide vector encodes a polypeptide comprising the amino acid sequence of SEQ ID NO:1, SEQ ID NO:2, or SEQ ID NO:3.
In other embodiments, the polynucleotide comprises a nucleic acid sequence having at least 70% identity to at least about 500 contiguous nucleotides selected from SEQ ID NO:4, SEQ ID NO:5, or SEQ ID NO:6, or the complement thereof. In some embodiments, the polynucleotide comprises a nucleic acid sequence having at least 70% identity to at least about 500, 600, 700, 800, 900, 1000, 1100, 1200, 1500, 2000, or 2400 contiguous nucleotides selected from SEQ ID NO:4, SEQ ID NO:5, or SEQ ID NO:6. In some embodiments, the polynucleotide comprises a nucleic acid sequence having at least 75% identity to at least about 500 contiguous nucleotides selected from SEQ ID NO:4, SEQ ID NO:5, or SEQ ID NO:6. In some embodiments, the polynucleotide comprises a nucleic acid sequence having at least 75% identity to at least about 500, 600, 700, 800, 900, 1000, 1100, 1200, 1500, 2000, or 2400 contiguous nucleotides selected from SEQ ID NO:4, SEQ ID NO:5, or SEQ ID NO:6. In some embodiments, the polynucleotide comprises a nucleic acid sequence having at least 80% identity to at least about 500 contiguous nucleotides selected from SEQ ID NO:4, SEQ ID NO:5, or SEQ ID NO:6. In some embodiments, the polynucleotide comprises a nucleic acid sequence having at least 80% identity to at least about 500, 600, 700, 800, 900, 1000, 1100, 1200, 1500, 2000, or 2400 contiguous nucleotides selected from SEQ ID NO:4, SEQ ID NO:5, or SEQ ID NO:6. In some embodiments, the polynucleotide comprises a nucleic acid sequence having at least 85% identity to at least about 500 contiguous nucleotides selected from SEQ ID NO:4, SEQ ID NO:5, or SEQ ID NO:6. In some embodiments, the polynucleotide comprises a nucleic acid sequence having at least 85% identity to at least about 500, 600, 700, 800, 900, 1000, 1100, 1200, 1500, 2000, or 2400 contiguous nucleotides selected from SEQ ID NO:4, SEQ ID NO:5, or SEQ ID NO:6. In some embodiments, the polynucleotide comprises a nucleic acid sequence having at least 90% identity to at least about 500 contiguous nucleotides selected from SEQ ID NO:4, SEQ ID NO:5, or SEQ ID NO:6. In some embodiments, the polynucleotide comprises a nucleic acid sequence having at least 90% identity to at least about 500, 600, 700, 800, 900, 1000, 1100, 1200, 1500, 2000, or 2400 contiguous nucleotides selected from SEQ ID NO:4, SEQ ID NO:5, or SEQ ID NO:6. In some embodiments, the polynucleotide comprises a nucleic acid sequence having at least 95% identity to at least about 500 contiguous nucleotides selected from SEQ ID NO:4, SEQ ID NO:5, or SEQ ID NO:6. In some embodiments, the polynucleotide comprises a nucleic acid sequence having at least 95% identity to at least about 500, 600, 700, 800, 900, 1000, 1100, 1200, 1500, 2000, or 2400 contiguous nucleotides selected from SEQ ID NO:4, SEQ ID NO:5, or SEQ ID NO:6. In certain embodiments, the polynucleotide comprises at least about 500 nucleotides selected from the nucleic acid sequence of SEQ ID NO:4, SEQ ID NO:5, or SEQ ID NO:6, or the complement thereof. In certain embodiments, the polynucleotide comprises at least about 500, 600, 700, 800, 900, 1000, 1100, 1200, 1500, 2000, or 2400 nucleotides selected from the nucleic acid sequence of SEQ ID NO:4, SEQ ID NO:5, or SEQ ID NO:6, or the complement thereof. In a particular embodiment, the polynucleotide comprises the nucleic acid sequence of SEQ ID NO:4, SEQ ID NO:5, or SEQ ID NO:6, or the complement thereof.

In one embodiment, the subject in the disclosed methods has a NYHA Class III heart failure. In another embodiment, the subject has a NYHA Class IV heart failure. In yet another embodiment, the subject has a plasma level of N-terminal pro-brain natriuretic peptide (NT-proBNP) of more than 4000 fmol/ml. In yet another embodiment, the subject is not responsive to neuregulin treatment.

In some embodiments, the polynucleotide vector is a viral vector. In a further embodiment, the viral vector is an adeno-associated viral vectors (AAV). In yet another embodiment, the AAV is adeno-associated viral vectors 9 (AAV9).

In some embodiments, the dose in the disclosed methods is about 1 x 10¹⁰-1 x 10¹⁵gc/patient. In some embodiments, the dose is about 1 x 10¹⁰-5 x 10¹⁴gc/patient. In some embodiments, the dose is about 1 x 10¹¹-1 x 10¹⁴gc/patient. In some embodiments, the dose is about 1 x 10¹¹-5 x 10¹³gc/patient. In some embodiments, the dose is about 5 x 10¹⁰-5 x 10¹¹ gc/patient. In some embodiment, the dose is about 2 x 10¹¹-5 x 10¹¹ gc/patient. In one embodiment, the dose is about 1 x 10¹⁰ gc/patient. In another embodiment, the dose is about 2 x 10¹⁰ gc/patient. In another embodiment, the dose is about 5 x 10¹⁰ gc/patient. In one embodiment, the dose is about 1 x 10¹¹ gc/patient. In another embodiment, the dose is about 2 x 10¹¹ gc/patient. In another embodiment, the dose is about 5 x 10¹¹ gc/patient. In one embodiment, the dose is about 1 x 10¹² gc/patient. In another embodiment, the dose is about 2 x 10¹² gc/patient. In another embodiment, the dose is about 5 x 10¹² gc/patient. In one embodiment, the dose is about 1 x 10¹³ gc/patient. In another embodiment, the dose is about 2 x 10¹³ gc/patient. In another embodiment, the dose is about 5 x 10¹³ gc/patient. In one embodiment, the dose is about 1 x 10¹⁴ gc/patient. In another embodiment, the dose is about 2 x 10¹⁴ gc/patient. In yet another embodiment, the dose is about 5 x 10¹⁴ gc/patient.

In another aspect, the present disclosure provides compositions for cMLCK gene therapy. In some embodiments, the composition is a polynucleotide vector that encodes a cMLCK protein. In some embodiments, the polynucleotide vector is a viral vector. In one embodiment, the viral vector is an adeno-associated viral vectors (AAV). In a specific embodiment, the polynucleotide vector is an AAV9.

In another aspect, the present disclosure provides pharmaceutical compositions. Such compositions comprise a therapeutically effective amount of a cMLCK-encoding polynucleotide vector and a pharmaceutically acceptable carrier. In some embodiments, the polynucleotide vector is a viral vector. In one embodiment, the viral vector is an adeno-associated viral vectors (AAV). In a specific embodiment, the polynucleotide vector is an AAV9. In some embodiments, water is the pharmaceutically acceptable carrier. In some embodiments, saline solutions and aqueous dextrose and glycerol solutions are employed as liquid carriers, particularly for injectable solutions.

In another aspect, the present disclosure provides compositions disclosed herein for use in the various disclosed methods. In some embodiments, the compositions disclosed herein are used for treating advanced heart failure in a subject, comprising administering to the subject an effective amount of a polynucleotide vector that encodes a cMLCK protein, expressing the cMLCK protein from the polynucleotide vector in the heart cells of the subject, and improving ventricular function of the heart. In some embodiments, the composition is a polynucleotide vector that encodes a cMLCK protein. In some embodiments, the polynucleotide vector is a viral vector. In one embodiment, the viral vector is an adeno-associated viral vectors (AAV). In a specific embodiment, the polynucleotide vector is an AAV9.

In other embodiments, the compositions disclosed herein are used for expressing a cMLCK protein in a subject, comprising administering to the subject a polynucleotide vector that encodes the cMLCK protein and expressing the cMLCK protein from the polynucleotide vector in the heart cells of the subject, wherein the subject has advanced heart failure. In some embodiments, the composition is a polynucleotide vector that encodes a cMLCK protein. In some embodiments, the polynucleotide vector is a viral vector. In one embodiment, the viral vector is an adeno-associated viral vectors (AAV). In a specific embodiment, the polynucleotide vector is an AAV9.

### BRIEF DESCRIPTION OF THE DRAWINGS

**FIG. 1** shows the genome structure of AAV9.cMLCK. A cMLCK-encoding polynucleotide (SEQ ID NO:4) was constructed into an AAV9 vector. ITR: inverted terminal repeat; CMV: cytomegalovirus; SVpA, poly(A) from SV40 viral genome.
**FIG. 2** shows the construction and quality control of cis plasmid pZac2.1-cMLCK. (A) Plasmid map of pZac2.1-cMLCK. (B) Restrictive enzyme digestion of pZac2.1-cMLCK. (C) Western blotting of cMLCK transgene expression after pZac2.1-cMLCK transfection into HEK293 cells.
**FIG. 3** shows the cMLCK transgene expression in (A) HEK293 cells and (B) isolated cardiac myocytes. Different dosages of AAV9.cMLCK were used to infect cells and cardiac myocytes. GAPDH was used as an internal control.
**FIG. 4** shows the quantification of cMLCK transgene mRNA expression in LV samples 5 weeks after indirect coronary gene transfer of AAV9.cMLCK. Indirect coronary injection was used to deliver different dosages of AAV9.cMLCK into mice. PBS was used as the control.
**FIG. 5** shows the echocardiography detection before and after AAV9.cMLCK gene transfer in pressure-overloaded mouse hearts. Transverse aortic constriction (TAC) was performed on mice. Three weeks after TAC, these mice were randomly divided into two groups. One group of mice received AAV9.cMLCK gene transfer by indirect intracoronary delivery, and another group of mice received PBS injection by the same way. Measurements were made three weeks after TAC and five weeks after AAV9.cMLCK gene transfer or PBS injection.
**FIG. 6** shows the *in vivo* hemodynamics determination after AAV9.cMLCK gene transfer in pressure-overloaded mouse hearts. Measurements were made five weeks after AAV9.cMLCK gene transfer or PBS injection.

### DETAILED DESCRIPTION OF THE INVENTION

The invention is based in part on the discovery that advanced heart failure patients are not responsive to neuregulin treatment. Within the scope of this invention, delivery of exogenous cMLCK to the heart of advanced heart failure patients and expression of the exogenous cMLCK therein is effective for improving cardiovascular function of the patients and treating heart failure. Thus, the present invention provides a treatment for advanced heart failure patients.

Unless defined otherwise, all technical and scientific terms used herein have the same meaning as is commonly understood by one of ordinary skill in the art to which this invention belongs. All patents, applications, published applications and other publications referred to herein are incorporated by reference in their entirety. If a definition set forth in this section is contrary to or otherwise inconsistent with a definition set forth in the patents, applications, published applications and other publications that are herein incorporated by reference, the definition set forth in this section prevails over the definition that is incorporated herein by reference.

As used herein, the singular forms "a," "an," and "the" mean "at least one" or "one or more" unless the context clearly dictates otherwise.

As used herein, "neuregulin" or "NRG" refers to proteins or peptides that can bind and activate ErbB2, ErbB3, ErbB4 or combinations thereof, including but not limited to all neuregulin isoforms, neuregulin EGF domain alone, polypeptides comprising neuregulin EGF-like domain, neuregulin mutants or derivatives, and any kind of neuregulin-like gene products that also activate the above receptors as described in detail below. Neuregulin can bind to and activates ErbB2/ErbB4 or ErbB2/ErbB3 heterodimers. Neuregulin also includes NRG-1, NRG-2, NRG-3, and NRG-4 proteins, peptides, fragments and compounds that mimic the activities of neuregulin. Neuregulin can activate the above ErbB receptors and modulate their biological reactions, *e.g.*, stimulate breast cancer cell differentiation and milk protein secretion; induce the differentiation of neural crest cell into Schwann cell; stimulate acetylcholine receptor synthesis in skeletal muscle cell; and/or improve cardiocyte differentiation, survival and DNA synthesis. Neuregulin also includes those variants with conservative amino acid substitutions that do not substantially alter their biological activity. Suitable conservative substitutions of amino acids are known to those of skill in this art and may be made generally without altering the biological activity of the resulting molecule. Those of skill in this art recognize that, in general, single amino acid substitutions in non-essential regions of a polypeptide do not substantially alter biological activity (*see, e.g.,* Watson et al., Molecular Biology of the Gene, 4th ed., The Benjamin/Cummings Pub. Co., p.224 (1987)). Neuregulin protein encompasses a neuregulin protein and peptide. Neuregulin nucleic acid encompasses neuregulin nucleic acid and neuregulin oligonucleotides.

As used herein, "cMLCK" refers to cardiac myosin light chain kinase, which include proteins or peptides which have an amino acid sequence that is identical to SEQ ID NO:1, SEQ ID NO:2, or SEQ ID NO:3, as well as proteins sharing sequence similarity, *e.g.*, 70%, 75%, 80%, 85%, 90%, 95%, or greater percent identity, with the amino acid sequence of SEQ ID NO:1, SEQ ID NO:2, or SEQ ID NO:3. Further, these proteins have a biological activity in common with the polypeptide having the amino acid sequence of SEQ ID NO:1, SEQ ID NO:2 or SEQ ID NO:3, including, but not limited to, antigenic cross-reactivity, autoinhibition, phosphorylation activity, and the like. It is also contemplated that a cMLCK protein can have one or more conservative or non-conservative amino acid substitutions, or additions or deletions from the amino acid sequence of SEQ ID NO:1, SEQ ID NO:2 or SEQ ID NO:3 so long as the protein having such sequence alteration shares a biological activity as described above with the polypeptide of SEQ ID NO:1, SEQ ID NO:2 or SEQ ID NO:3. cMLCK also includes proteins or peptides expressed from different mutations, different spliced forms and various sequence polymorphisms of the cMLCK gene. cMLCK includes its variants that maintain one or more of its functions of cMLCK. It is recognized that the gene or cDNA encoding cMLCK can be considerably mutated without materially altering one or more cMLCK functions. First, the genetic code is well-known to be degenerate, and thus different codons may encode the same amino acids. Second, even where an amino acid substitution is introduced, the mutation can be conservative and have no material impact on the essential functions of cMLCK. Third, part of the cMLCK polypeptide can be deleted without impairing or eliminating all of its functions. Fourth, insertions or additions can be made in cMLCK, for example, adding epitope tags, without impairing or eliminating its functions. Other modifications can be made without materially impairing one or more functions of cMLCK, for example, *in vivo* or *in vitro* chemical and biochemical modifications which incorporate unusual amino acids. Such modifications include, for example, acetylation, carboxylation, phosphorylation, glycosylation, ubiquination, labeling with radionuclides, and various enzymatic modifications, as will be readily appreciated by those skilled in the art. A variety of methods for labeling proteins and substituents or labels useful for such purposes are well known in the art, and include radioactive isotopes such as ligands which bind to labeled antiligands (*e.g.,* antibodies), fluorophores, chemiluminescent agents, enzymes, and antiligands. Functional fragments and variants can be of varying length. For example, some fragments have at least 10, 25, 50, 75, 100, or 200 or more amino acid residues.

As used herein, "functional fragments and variants of cMLCK" refer to those fragments and variants that maintain one or more functions of cMLCK. It is recognized that the gene or cDNA encoding cMLCK can be considerably mutated without materially altering one or more cMLCK functions. First, the genetic code is well-known to be degenerate, and thus different codons may encode the same amino acids. Second, even where an amino acid substitution is introduced, the mutation can be conservative and have no material impact on the essential functions of cMLCK. Third, part of the cMLCK polypeptide can be deleted without impairing or eliminating all of its functions. Fourth, insertions or additions can be made in cMLCK, for example, adding epitope tags, without impairing or eliminating its functions. Other modifications can be made without materially impairing one or more functions of cMLCK, for example, *in vivo* or *in vitro* chemical and biochemical modifications which incorporate unusual amino acids. Such modifications include, for example, acetylation, carboxylation, phosphorylation, glycosylation, ubiquination, labeling with radionuclides, and various enzymatic modifications, as will be readily appreciated by those skilled in the art. A variety of methods for labeling proteins and substituents or labels useful for such purposes are well known in the art, and include radioactive isotopes such as ligands which bind to labeled antiligands (*e.g.,* antibodies), fluorophores, chemiluminescent agents, enzymes, and antiligands. Functional fragments and variants can be of varying length. For example, some fragments have at least 10, 25, 50, 75, 100, or 200 or more amino acid residues.

As used herein, "myosin light chain" refers to an about 18 kDa protein which associates with the myosin heavy chain and participates in the regulation of myosin's force-generating ATPase activity. There are two major groupings of MLC: MLC1, sometimes referred to as the essential myosin light chain, abbreviated ELC; and MLC2, sometimes referred to as the regulatory myosin light chain, abbreviated RLC. RLC is the primary biological target of myosin light chain kinase (MLCK) -mediated phosphorylation. When phosphorylated by MLCK the phosphorylated form of RLC is abbreviated of RLC-P. Isoforms of ELC and RLC existing in skeletal, smooth, and cardiac muscle have been described. As an example, the human cardiac RLC gene and cDNA are described by Macera et al., Genomics 13: 829-831 (1992); (GenBank accession No. NM00432).

As used herein, a "functional fragment or variant of myosin light chain" refers to a polypeptide which is capable of being phosphorylated by a protein having myosin light chain kinase biological activity. It includes any polypeptide six or more amino acid residues in length which is capable of being phosphorylated by a protein having myosin light chain kinase biological activity.

As used herein, "myosin light chain kinase biological activity" refers to the *in vitro* or *in vivo* enzymatic ability of a polypeptide or protein to mediate covalent incorporation of a phosphate into a regulatory myosin light chain. The term encompasses such enzymatic activity observed with any isoform of MLCK (for example, smooth muscle, skeletal muscle, and cardiac MLCK isoforms), as well as such enzymatic activity observed with fragments and variants of MLCK isoforms (for example, naturally occurring mutants; mutations, insertions and deletions introduced through recombinant DNA techniques; and fragments generated by proteolysis).

As used herein, "protein" is synonymous with "polypeptide" or "peptide" unless the context clearly dictates otherwise.

As used herein, the terms "nucleic acid", "nucleotide" and "polynucleotide" refer to deoxyribonucleotides, deoxyribonucleic acids, ribonucleotides, and ribonucleic acids, and polymeric forms thereof, and include either single- or double-stranded forms. In some embodiments, such terms refer to deoxyribonucleic acids (*e.g.,* cDNA or DNA). In other embodiments, such terms refer to ribonucleic acid (*e.g.,* mRNA or RNA).

As used herein, the terms "subject" and "patient" are used interchangeably and refer to a mammal such as a non-primate (*e.g.,* cows, pigs, horses, cats, dogs, rats etc.) and a primate (*e.g.,* monkey and human), most preferably a human.

As used herein, "vector" refers to discrete elements that are used to introduce heterologous DNA into cells for either expression or replication thereof. Selection and use of such vehicles are well known within the skill of the artisan. An expression vector includes vectors capable of expressing DNA that are operatively linked with regulatory sequences, such as promoter regions, that are capable of effecting expression of such DNA fragments. Thus, an expression vector refers to a recombinant DNA or RNA construct, such as a plasmid, a phage, recombinant virus or other vector that, upon introduction into an appropriate host cell, results in expression of the cloned DNA. Appropriate expression vectors are well known to those of skill in the art and include those that are replicable in eukaryotic cells and/or prokaryotic cells and those that remain episomal or those which integrate into the host cell genome.

As used herein, "ejection fraction" or "EF" means the portion of blood that is pumped out of a filled left ventricle (LV) as the result of a heartbeat. It may be defined by the following formula: (LV diastolic volume - LV systolic volume) / LV diastolic volume.

As used herein, "fractional shortening" or "FS" means a ratio of the change in the diameter of the left ventricle between the contracted and relaxed states. It may be defined by the following formula: (LV end diastolic diameter - LV end systolic diameter) / LV end diastolic diameter.

As used herein, "heart failure" means an abnormality of cardiac function where the heart does not pump blood at the rate needed for the requirements of metabolizing tissues. Heart failure includes a wide range of disease states such as congestive heart failure, myocardial infarction, tachyarrhythmia, familial hypertrophic cardiomyopathy, ischemic heart disease, idiopathic dilated cardiomyopathy, myocarditis and the like. The heart failure can be caused by any number of factors, including, without limitation, ischemic, congenital, rheumatic, or idiopathic forms. Chronic cardiac hypertrophy is a significantly diseased state which is a precursor to congestive heart failure and cardiac arrest.

As used herein, "advanced heart failure" means a heart failure condition with at least one of the following characteristics: (1) disease progression to NYHA Class III and IV, especially Class IV, (2) episodes with clinical signs of fluid retention and/or peripheral hypoperfusion, (3) objective evidence of severe cardiac dysfunction, shown by at least one of the following: left ventricular ejection fraction of less than 30%, pseudonormal or restrictive mitral inflow pattern at Doppler-echocardiography; high left and/or right ventricular filling pressures; elevated plasma level of N-terminal pro-brain natriuretic peptide (NT-proBNP) or BNP, (4) severe impairment of functional capacity demonstrated by either inability to exercise, a 6-minute walk test distance of less than 300 m or a peak oxygen uptake of less than 12-14 ml/kg/min, (5) history of more than one heart failure hospitalization in the past 6 months, or (6) presence of any of the five previous characteristics despite conventional heart therapies and symptom management strategies.

It is well known in the art that a patient's heart failure can be classified according to the severity of their symptoms. The most commonly used classification system is the New York Heart Association (NYHA) Functional Classification. It places patients in one of four categories based on how much they are limited during physical activity, as follows: Class I: patients with cardiac disease but resulting in no limitation of physical activity. Ordinary physical activity does not cause undue fatigue, palpitation, dyspnea or anginal pain; Class II: patients with cardiac disease resulting in slight limitation of physical activity. They are comfortable at rest. Ordinary physical activity results in fatigue, palpitation, dyspnea or anginal pain; Class III: patients with cardiac disease resulting in marked limitation of physical activity. They are comfortable at rest. Less than ordinary activity causes fatigue, palpitation, dyspnea or anginal pain; Class IV: patients with cardiac disease resulting in inability to carry on any physical activity without discomfort. Symptoms of heart failure or the anginal syndrome may be present even at rest. If any physical activity is undertaken, discomfort increases.

As used herein, "N-terminal pro-brain natriuretic peptide" or "NT-proBNP" means the inactive remnant N-terminal proBNP, which is the pro hormone of BNP. BNP is a hormonally active natriuretic peptide that is mainly released from the cardiomyocytes in the left ventricular wall. In reaction to stretch and tension of the myocardial wall, proBNP splits into BNP and the hormonally inactive remnant NT-proBNP by proteolytic cleavage. The plasma NT-proBNP level can be analyzed by commercial kits. Different kits may have different results regarding the same sample; however, the results of different kits can to conversed into each other. The numerical value of plasma NT-proBNP level in the present disclosure is measured using the commercial kit by Biomedica, Austria (CE No. Q1530510). As used herein, an "effective amount" of an active agent for treating a particular disease is an amount that is sufficient to ameliorate, or in some manner reduce the symptoms associated with the disease. The amount may cure the disease but, typically, is administered in order to ameliorate the symptoms of the disease.

The terms "treatment," "treating," and the like are used herein to generally mean obtaining a desired pharmacological and/or physiological effect in a subject actively suffering from a condition. The effect may completely or partially treat a disease or symptom thereof and thus may be therapeutic in terms of a partial or complete cure for a disease and/or adverse effect attributable to the disease. "Treatment" as used herein covers any treatment of a disease in a mammal, particularly a human, and includes inhibiting the disease, *i.e.,* arresting its development; or relieving the disease, *i.e.,* causing regression of the disease. In one example, treatment refers to treating patients with, or at risk for, development of heart disease and related conditions, *e.g.*, advanced heart failure. More specifically, "treatment" is intended to mean providing a therapeutically detectable and beneficial effect on a patient suffering from heart disease.

The terms "prevent," "preventing," and the like are used herein to generally refer to preventing a disease from occurring in a subject which may be predisposed to the disease but has not yet been diagnosed as suffering from the disease. Thus, "prevent" can refer to prophylactic or preventative measures, wherein the object is to prevent or slow down (lessen) cardiac hypertrophy.

As used herein, "recombinant expression vector" refers to systems of polynucleotides which operatively encode polypeptides expressible in eukaryotes or prokaryotes.
As used herein, the term "pharmaceutically acceptable" means approved by a regulatory agency of the Federal or a state government, or listed in the U.S. Pharmacopeia or other generally recognized pharmacopeia for use in animals, and more particularly in humans.

As used herein, the term "carrier" refers to a diluent, adjuvant, excipient, or vehicle with which a therapeutic of the invention is administered. Such pharmaceutical carriers can be sterile liquids, such as water and oils, including those of petroleum, animal, vegetable or synthetic origin, such as peanut oil, soybean oil, mineral oil, sesame oil and the like.

In some aspects, the present disclosure provides methods for treating advanced heart failure in a subject, comprising administering to the subject an effective amount of a polynucleotide vector that encodes a cMLCK protein, expressing the cMLCK protein from the polynucleotide vector in the heart cells of the subject, and improving ventricular function of the heart. In some embodiments, the subject is a human.

In some aspects, the present disclosure provides methods for expressing a cMLCK protein in a subject, comprising administering to the subject a polynucleotide vector that encodes the cMLCK protein and expressing the cMLCK protein from the polynucleotide vector in the heart cells of the subject, wherein the subject has advanced heart failure. In some embodiments, the subject is a human. In some embodiments, the subject has an improvement in ventricular function of the heart.

In some embodiments, the cMLCK-encoding polynucleotide vector is systemically administered to the subject. In other embodiments, the cMLCK-encoding polynucleotide vector is locally administered to the subject. Methods of administration include but are not limited to intravenous, intracoronary, and intraventricular. The polynucleotide vector may be administered by any convenient route, for example by infusion or bolus injection, and may be administered together with other biologically active agents.

In some specific embodiments, it may be desirable to administer the cMLCK-encoding polynucleotide vector locally to the heart of the subject. This may be achieved by, for example, and not by way of limitation, intracoronary infusion, local infusion during surgery, by injection, by means of a catheter, or by means of an implant, said implant being of a porous, non-porous, or gelatinous material, including membranes, such as sialastic membranes, or fibers. Intracoronary infusion may be facilitated by an intracoronary catheter, for example, attached to a pump. In some embodiments, the cMLCK-encoding polynucleotide vector is locally administered into the subject's heart via intracoronary infusion. In some embodiments, the cMLCK-encoding polynucleotide vector is locally administered into the subject's LV cavity. In other embodiments, the cMLCK-encoding polynucleotide vector is locally administered into the subject's heart surgically (*i.e.,* by direct introduction of the vector or transfected cells into the subject's heart).

In some embodiments, the cMLCK-encoding polynucleotide vector is administered to the heart of the subject in a liposome. Various delivery systems are known and can be used to administer cMLCK-encoding polynucleotide vector in the invention, *e.g.*, encapsulation in liposomes; microparticles; microcapsules; receptor-mediated endocytosis (*see, e.g.,* Wu and Wu, J. Biol. Chem. 262:4429-4432 (1987)); micells biocompatible polymers, including natural polymers and synthetic polymers; lipoproteins; polypeptides; polysaccharides; lipopolysaccharides; artificial viral envelopes; metal particles; and bacteria, or viruses, such as baculovirus, adenovirus and retrovirus, bacteriophage, cosmid, plasmid, fungal vectors and other recombination vehicles typically used in the art. In a specific embodiment, the cMLCK-encoding polynucleotide can be delivered in a vesicle, in particular, a liposome (*see* Langer, Science 249:1527-1533 (1990); Treat et al., in Liposomes in the Therapy of Infectious Disease and Cancer, Lopez-Berestein and Fidler (eds.), Liss, New York, pp. 317-372, 353-365 (1989)).

The subject in the disclosed methods is assessed for improvements of ventricular function of the heart, using a number of parameters, including without limitation, EF values of the left ventricle, Left Ventricular End-Diastolic Volume (LVEDV), Left Ventricular End-Systolic Volume (LVESV), Left Ventricular End-Diastolic Pressure (LVEDP), Left Ventricular Systolic Pressure (LVSP). These parameters may be monitored using methods well-known in the art, for example, by echocardiography. In some embodiments, the EF values of the left ventricle of the subject is enhanced by at least 5%. In some embodiments, the EF values of the left ventricle of the subject is enhanced by at least 10%. In some embodiments, the EF values of the left ventricle of the subject is enhanced by at least 20%. In some embodiments, the LVEDV or LVESV of the subject is reduced by at least 5%. In some embodiments, the LVEDV or LVESV of the subject is reduced by at least 10%. In some embodiments, the LVEDV or LVESV of the subject is reduced by at least 20%.

The polynucleotide vector encoding cMLCK include those polynucleotide vectors encoding substantially the same amino acid sequences as found in native cMLCK, as well as those encoding amino acid sequences having functionally inconsequential amino acid substitutions, and thus have amino acid sequences which differ from that of the native sequence. Examples include the substitution of one basic residue for another (*e.g.,* Arg for Lys), the substitution of one hydrophobic residue for another (*e.g.,* Leu for Ile), or the substitution of one aromatic residue for another (*e.g.,* Phe for Tyr).

The polynucleotide vector encoding cMLCK also include fragments of cMLCK. The polynucleotide of the invention include human and related genes (homologues) in other species. In some embodiments, the polynucleotide sequences are from vertebrates, or more particularly, mammals. In some embodiments, the polynucleotide sequences are of rat origin. In some embodiments of the invention, the polynucleotide sequences are of human origin.

In some embodiments, the polynucleotide vector encodes a polypeptide comprising an amino acid sequence having at least 70% identity to SEQ ID NO:1. In some embodiments, the polynucleotide vector encodes a polypeptide comprising an amino acid sequence having at least 75%, 80%, 85%, 90%, or 95% identity to SEQ ID NO:1. In a particular embodiment, the polynucleotide vector encodes a polypeptide comprising the amino acid sequence of SEQ ID NO:1.

In other embodiments, the polynucleotide comprises a nucleic acid sequence having at least 70% identity to at least about 500 contiguous nucleotides selected from SEQ ID NO:4 or the complement thereof. In some embodiments, the polynucleotide comprises a nucleic acid sequence having at least 70% identity to at least about 500, 600, 700, 800, 900, 1000, 1100, 1200, 1500, 2000, or 2400 contiguous nucleotides selected from SEQ ID NO:4. In some embodiments, the polynucleotide comprises a nucleic acid sequence having at least 75% identity to at least about 500 contiguous nucleotides selected from SEQ ID NO:4. In some embodiments, the polynucleotide comprises a nucleic acid sequence having at least 75% identity to at least about 500, 600, 700, 800, 900, 1000, 1100, 1200, 1500, 2000, or 2400 contiguous nucleotides selected from SEQ ID NO:4. In some embodiments, the polynucleotide comprises a nucleic acid sequence having at least 80% identity to at least about 500 contiguous nucleotides selected from SEQ ID NO:4. In some embodiments, the polynucleotide comprises a nucleic acid sequence having at least 80% identity to at least about 500, 600, 700, 800, 900, 1000, 1100, 1200, 1500, 2000, or 2400 contiguous nucleotides selected from SEQ ID NO:4. In some embodiments, the polynucleotide comprises a nucleic acid sequence having at least 85% identity to at least about 500 contiguous nucleotides selected from SEQ ID NO:4. In some embodiments, the polynucleotide comprises a nucleic acid sequence having at least 85% identity to at least about 500, 600, 700, 800, 900, 1000, 1100, 1200, 1500, 2000, or 2400 contiguous nucleotides selected from SEQ ID NO:4. In some embodiments, the polynucleotide comprises a nucleic acid sequence having at least 90% identity to at least about 500 contiguous nucleotides selected from SEQ ID NO:4. In some embodiments, the polynucleotide comprises a nucleic acid sequence having at least 90% identity to at least about 500, 600, 700, 800, 900, 1000, 1100, 1200, 1500, 2000, or 2400 contiguous nucleotides selected from SEQ ID NO:4. In some embodiments, the polynucleotide comprises a nucleic acid sequence having at least 95% identity to at least about 500 contiguous nucleotides selected from SEQ ID NO:4. In some embodiments, the polynucleotide comprises a nucleic acid sequence having at least 95% identity to at least about 500, 600, 700, 800, 900, 1000, 1100, 1200, 1500, 2000, or 2400 contiguous nucleotides selected from SEQ ID NO:4. In certain embodiments, the polynucleotide comprises at least about 500 nucleotides selected from the nucleic acid sequence of SEQ ID NO:4, or the complement thereof. In certain embodiments, the polynucleotide comprises at least about 500, 600, 700, 800, 900, 1000, 1100, 1200, 1500, 2000, or 2400 nucleotides selected from the nucleic acid sequence of SEQ ID NO:4, or the complement thereof. In a particular embodiment, the polynucleotide comprises the nucleic acid sequence of SEQ ID NO:4, or the complement thereof.

In some embodiments, the polynucleotide vector encodes a polypeptide comprising an amino acid sequence having at least 70% identity to SEQ ID NO:2. In some embodiments, the polynucleotide vector encodes a polypeptide comprising an amino acid sequence having at least 75%, 80%, 85%, 90%, or 95% identity to SEQ ID NO:2. In a particular embodiment, the polynucleotide vector encodes a polypeptide comprising the amino acid sequence of SEQ ID NO:2.

In other embodiments, the polynucleotide comprises a nucleic acid sequence having at least 70% identity to at least about 500 contiguous nucleotides selected from SEQ ID NO:5 or the complement thereof. In some embodiments, the polynucleotide comprises a nucleic acid sequence having at least 70% identity to at least about 500, 600, 700, 800, 900, 1000, 1100, 1200, 1500, 2000, or 2400 contiguous nucleotides selected from SEQ ID NO:5. In some embodiments, the polynucleotide comprises a nucleic acid sequence having at least 75% identity to at least about 500 contiguous nucleotides selected from SEQ ID NO:5. In some embodiments, the polynucleotide comprises a nucleic acid sequence having at least 75% identity to at least about 500, 600, 700, 800, 900, 1000, 1100, 1200, 1500, 2000, or 2400 contiguous nucleotides selected from SEQ ID NO:5. In some embodiments, the polynucleotide comprises a nucleic acid sequence having at least 80% identity to at least about 500 contiguous nucleotides selected from SEQ ID NO:5. In some embodiments, the polynucleotide comprises a nucleic acid sequence having at least 80% identity to at least about 500, 600, 700, 800, 900, 1000, 1100, 1200, 1500, 2000, or 2400 contiguous nucleotides selected from SEQ ID NO:5. In some embodiments, the polynucleotide comprises a nucleic acid sequence having at least 85% identity to at least about 500 contiguous nucleotides selected from SEQ ID NO:5. In some embodiments, the polynucleotide comprises a nucleic acid sequence having at least 85% identity to at least about 500, 600, 700, 800, 900, 1000, 1100, 1200, 1500, 2000, or 2400 contiguous nucleotides selected from SEQ ID NO:5. In some embodiments, the polynucleotide comprises a nucleic acid sequence having at least 90% identity to at least about 500 contiguous nucleotides selected from SEQ ID NO:5. In some embodiments, the polynucleotide comprises a nucleic acid sequence having at least 90% identity to at least about 500, 600, 700, 800, 900, 1000, 1100, 1200, 1500, 2000, or 2400 contiguous nucleotides selected from SEQ ID NO:5. In some embodiments, the polynucleotide comprises a nucleic acid sequence having at least 95% identity to at least about 500 contiguous nucleotides selected from SEQ ID NO:5. In some embodiments, the polynucleotide comprises a nucleic acid sequence having at least 95% identity to at least about 500, 600, 700, 800, 900, 1000, 1100, 1200, 1500, 2000, or 2400 contiguous nucleotides selected from SEQ ID NO:5. In certain embodiments, the polynucleotide comprises at least about 500 nucleotides selected from the nucleic acid sequence of SEQ ID NO:5, or the complement thereof. In certain embodiments, the polynucleotide comprises at least about 500, 600, 700, 800, 900, 1000, 1100, 1200, 1500, 2000, or 2400 nucleotides selected from the nucleic acid sequence of SEQ ID NO:5, or the complement thereof. In a particular embodiment, the polynucleotide comprises the nucleic acid sequence of SEQ ID NO:5, or the complement thereof.

In some embodiments, the polynucleotide vector encodes a polypeptide comprising an amino acid sequence having at least 70% identity to SEQ ID NO:3. In some embodiments, the polynucleotide vector encodes a polypeptide comprising an amino acid sequence having at least 75%, 80%, 85%, 90%, or 95% identity to SEQ ID NO:3. In a particular embodiment, the polynucleotide vector encodes a polypeptide comprising the amino acid sequence of SEQ ID NO:3.

In other embodiments, the polynucleotide comprises a nucleic acid sequence having at least 70% identity to at least about 500 contiguous nucleotides selected from SEQ ID NO:6 or the complement thereof. In some embodiments, the polynucleotide comprises a nucleic acid sequence having at least 70% identity to at least about 500, 600, 700, 800, 900, 1000, 1100, 1200, 1500, 2000, or 2400 contiguous nucleotides selected from SEQ ID NO:6. In some embodiments, the polynucleotide comprises a nucleic acid sequence having at least 75% identity to at least about 500 contiguous nucleotides selected from SEQ ID NO:6. In some embodiments, the polynucleotide comprises a nucleic acid sequence having at least 75% identity to at least about 500, 600, 700, 800, 900, 1000, 1100, 1200, 1500, 2000, or 2400 contiguous nucleotides selected from SEQ ID NO:6. In some embodiments, the polynucleotide comprises a nucleic acid sequence having at least 80% identity to at least about 500 contiguous nucleotides selected from SEQ ID NO:6. In some embodiments, the polynucleotide comprises a nucleic acid sequence having at least 80% identity to at least about 500, 600, 700, 800, 900, 1000, 1100, 1200, 1500, 2000, or 2400 contiguous nucleotides selected from SEQ ID NO:6. In some embodiments, the polynucleotide comprises a nucleic acid sequence having at least 85% identity to at least about 500 contiguous nucleotides selected from SEQ ID NO:6. In some embodiments, the polynucleotide comprises a nucleic acid sequence having at least 85% identity to at least about 500, 600, 700, 800, 900, 1000, 1100, 1200, 1500, 2000, or 2400 contiguous nucleotides selected from SEQ ID NO:6. In some embodiments, the polynucleotide comprises a nucleic acid sequence having at least 90% identity to at least about 500 contiguous nucleotides selected from SEQ ID NO:6. In some embodiments, the polynucleotide comprises a nucleic acid sequence having at least 90% identity to at least about 500, 600, 700, 800, 900, 1000, 1100, 1200, 1500, 2000, or 2400 contiguous nucleotides selected from SEQ ID NO:6. In some embodiments, the polynucleotide comprises a nucleic acid sequence having at least 95% identity to at least about 500 contiguous nucleotides selected from SEQ ID NO:6. In some embodiments, the polynucleotide comprises a nucleic acid sequence having at least 95% identity to at least about 500, 600, 700, 800, 900, 1000, 1100, 1200, 1500, 2000, or 2400 contiguous nucleotides selected from SEQ ID NO:6. In certain embodiments, the polynucleotide comprises at least about 500 nucleotides selected from the nucleic acid sequence of SEQ ID NO:6, or the complement thereof. In certain embodiments, the polynucleotide comprises at least about 500, 600, 700, 800, 900, 1000, 1100, 1200, 1500, 2000, or 2400 nucleotides selected from the nucleic acid sequence of SEQ ID NO:6, or the complement thereof. In a particular embodiment, the polynucleotide comprises the nucleic acid sequence of SEQ ID NO:6, or the complement thereof.

Cloning of cMLCK-encoding polynucleotides is disclosed in WO 08/28405, the contents of which are incorporated by reference. The polynucleotides may be obtained by standard procedures known in the art from cloned DNA (*e.g.,* a DNA "library"), by chemical synthesis, by cDNA cloning, or by the cloning of genomic DNA, or fragments thereof, purified from the desired cell, or by PCR amplification and cloning. *See, e.g.,* Sambrook et al., Molecular Cloning, A Laboratory Manual, 3d. ed., Cold Spring Harbor Laboratory Press, Cold Spring Harbor, New York (2001); Glover, D.M. (ed.), DNA Cloning: A Practical Approach, 2d. ed., MRL Press, Ltd., Oxford, U.K. (1995). Clones derived from genomic DNA may contain regulatory and intron DNA regions in addition to coding regions; clones derived from cDNA will contain only exon sequences. Whatever the source, the gene may be cloned into a suitable vector for propagation of the gene.

The cMLCK-encoding polynucleotides can then be inserted into an appropriate cloning vector. A large number of vector-host systems known in the art may be used. Possible cloning vectors include, but are not limited to, plasmids or modified viruses. Techniques for construction of expression vectors are well known in the art. *See, e.g.,* Sambrook et al., 2001, Molecular Cloning - A Laboratory Manual, 3rd edition, Cold Spring Harbor Laboratory, Cold Spring Harbor, NY, and Ausubel et al., eds., Current Edition, Current Protocols in Molecular Biology, Greene Publishing Associates and Wiley Interscience, NY. The insertion into a cloning vector can, for example, be accomplished by ligating the DNA fragment into a cloning vector which has complementary cohesive termini. However, if the complementary restriction sites used to fragment the DNA are not present in the cloning vector, the ends of the DNA molecules may be enzymatically modified. Alternatively, any site desired may be produced by ligating nucleotide sequences (linkers) onto the DNA termini. These ligated linkers may comprise specific chemically synthesized oligonucleotides encoding restriction endonuclease recognition sequences. In an alternative method, the cleaved vector and cMLCK-encoding polynucleotide sequence may be modified by homopolymeric tailing.

In one embodiment, the subject in the disclosed methods has a NYHA Class III heart failure. In another embodiment, the subject has a NYHA Class IV heart failure. NYHA Class III heart failure and NYHA Class IV heart failure are defined above.

In yet another embodiment, the subject has a plasma level of N-terminal pro-brain natriuretic peptide (NT-proBNP) of more than 4000 fmol/ml. BNP and NT-proBNP plasma levels are promising tools in the daily management of suspected or established heart failure. Most studies on the use of BNP and NT-proBNP in clinical practice addressed their diagnostic properties, and an increasingly amount of evidence is available supporting the prognostic value of BNP and NT-proBNP. As NT-proBNP has about 6 times longer of half life in the blood than BNP, it is more widely used as a diagnostic or prognostic marker for heart failure. The plasma NT-proBNP level can be analyzed by commercial kits. Different kits may have different results regarding the same sample; however, the results of different kits can to conversed into each other.

Both BNP and NT-proBNP levels in the blood are used for screening and diagnosis of heart failure and are useful to establish prognosis in heart failure, as both markers are typically higher in patients with worse outcome. Thus, it is discovered a significantly elevated plasma level of BNP or NT-proBNP is indicative of the patient being suitable for heart failure treatment by cMLCK gene transfer. The compositions and methods of the invention are suitable for human patient with advanced heart failure, for example, the patient has a plasma level of N-terminal pro-brain natriuretic peptide (NT-proBNP) of more than 4000 fmol/ml, as measured using the commercial kit by Biomedica, Austria (CE No. Q1530510).

In yet another embodiment, the subject is not responsive to neuregulin treatment. The subject is not responsive to neuregulin treatment when neuregulin is not effective in ameliorating, or in some manner reducing the symptoms associated with the condition. Whether the subject is responsive to neuregulin treatment can be determined by standard clinical techniques well known to those of skill in the art, including, but not limited to, by examining the symptoms or parameters associated with the condition, such as NYHA Classification, EF value of the left ventricle, LVESV, LVEDV, 6 Minute Walk Distance, plasma level of BNP or NT-proBNP, Quality of Life Analysis (Kansas City Cardiomyopathy Questionnaire), all cause mortality, and all cause hospitalization.

In some embodiments, the polynucleotide vector is a viral vector. In a further embodiment, the viral vector is an adeno-associated viral vectors (AAV). In yet another embodiment, the AAV is adeno-associated viral vectors 9 (AAV9).

Gene delivery, gene transfer, transducing, and the like as used herein, are terms referring to the introduction of an exogenous polynucleotide (sometimes referred to as a transgene) into a host cell, irrespective of the method used for the introduction. Such methods include a variety of well-known techniques such as techniques facilitating the delivery of naked polynucleotides (such as electroporation, "gene gun" delivery and various other techniques used for the introduction of polynucleotides), as well as vector-mediated gene transfer (by, *e.g.,* viral infection/transfection, or various other protein-based or lipid-based gene delivery complexes). The introduced polynucleotide can be stably or transiently maintained in the host cell. Stable maintenance typically requires that the introduced polynucleotide either contains an origin of replication compatible with the host cell or integrates into a replicon of the host cell such as an extrachromosomal replicon (*e.g.,* a plasmid) or a nuclear or mitochondrial chromosome. A number of vectors are known to be capable of mediating transfer of genes to mammalian cells, as is known in the art.

A viral vector refers to a recombinantly produced virus or viral particle that comprises a polynucleotide to be delivered into a host cell. Examples of viral vectors include RNA virus such as retroviral vectors, herpes virus vectors, vaccinia vectors, adenovirus vectors, adeno-associated virus vectors, alphavirus vectors and the like. Alphavirus vectors, such as Semliki Forest virus-based vectors and Sindbis virus-based vectors, have also been developed for use in gene therapy and immunotherapy (*see* Schlesinger and Dubensky, Curr. Opin. Biotechnol. 5:434-439 (1999) and Ying, et al. Nat. Med. 5(7):823-827 (1999)).

In embodiments where gene transfer is mediated by a retroviral vector, a vector construct refers to the polynucleotide comprising the retroviral genome or part thereof, and a therapeutic gene. As used herein, retroviral mediated gene transfer or retroviral transduction carries the same meaning and refers to the process by which a gene or nucleic acid sequences are stably transferred into the host cell by virtue of the virus entering the cell and integrating its genome into the host cell genome. The virus can enter the host cell via its normal mechanism of infection or be modified such that it binds to a different host cell surface receptor or ligand to enter the cell. As used herein, retroviral vector refers to a viral particle capable of introducing exogenous nucleic acid into a cell through a viral or viral-like entry mechanism. Retroviruses carry their genetic information in the form of RNA; however, once the virus infects a cell, the RNA is reverse-transcribed into the DNA form which integrates into the genomic DNA of the infected cell. The integrated DNA form is called a provirus. Examples of retroviral vectors in which a single foreign gene can be inserted include, but are not limited to, Moloney murine leukemia virus (MoMuLV) vectors, harvey murine sarcoma virus (HaMuSV) vectors, murine mammary tumor virus (MuMTV) vectors, and Rous Sarcoma Virus (RSV) vectors. A number of additional retroviral vectors can incorporate a gene for a selectable marker so that transduced cells can be identified and generated.

In embodiments where gene transfer is mediated by a DNA viral vector, such as an adenovirus (Ad) or adeno-associated virus (AAV), a vector construct refers to the polynucleotide comprising the viral genome or part thereof, and a transgene. Adenoviruses (Ads) are a relatively well characterized, homogenous group of viruses, including over 50 serotypes (*see, e.g.,* WO 95/27071). Ads do not require integration into the host cell genome. Recombinant Ad derived vectors, particularly those that reduce the potential for recombination and generation of wild-type virus, have also been constructed (*see, e.g.,* WO 95/00655 and WO 95/11984).

Wild-type AAV has high infectivity and specificity integrating into the host cell's genome (*see, e.g.,* Hermonat and Muzyczka, Proc. Natl. Acad. Sci. USA 81:6466-6470 (1984) and Lebkowski et al., Mol. Cell. Biol. 8:3988-3996 (1988)). As used herein, adeno-associated virus or AAV encompasses all subtypes, serotypes and pseudotypes, as well as naturally occurring and recombinant forms. A variety of AAV serotypes and strains are known in the art and are publicly available from sources, such as the ATCC, and academic or commercial sources. Alternatively, sequences from AAV serotypes and strains which are published and/or available from a variety of databases may be synthesized using known techniques. Adeno-associated viruses (AAV) are parvoviruses belonging to the genus Dependovirus. They are small, nonenveloped, single-stranded DNA viruses that require a helper virus in order to replicate. Co-infection with a helper virus (*e.g.,* adenovirus, herpes virus, or vaccinia virus) is necessary to form functionally complete AAV virions. *In vitro,* in the absence of co-infection with a helper virus, AAV establishes a latent state in which the viral genome exists in an episomal form, but infectious virions are not produced. Subsequent infection by a helper virus "rescues" the genome, allowing it to be replicated and packaged into viral capsids, thereby reconstituting the infectious virion. Recent data indicate that *in vivo* both wild type AAV and recombinant AAV predominantly exist as large episomal concatemers. AAVs are not associated with any known human diseases, are generally not considered pathogenic, and do not appear to alter the physiological properties of the host cell upon integration. AAV can infect a wide range of host cells, including non-dividing cells, and can infect cells from different species. In contrast to some vectors, which are quickly cleared or inactivated by both cellular and humoral responses. AAV vectors have shown persistent expression in various tissues *in vivo.* The persistence of recombinant AAV vectors in non-diving cells *in vivo* may be attributed to the lack of native AAV viral genes and the vector's ability to form episomal concatemers.

Andeno-associated virus (AAV) is an attractive vector system for use in the cell transduction of the present disclosure as it has a high frequency of persistence as an episomal concatemer and it can infect non-dividing cells, thus making it useful for delivery of genes into mammalian cells, for example, in tissue culture and *in vivo.* Studies demonstrating the use of AAV in gene delivery include Flotte et al., Proc. Natl. Acad. Sci. USA 90:10613-10617 (1993) and Walsh et al,, J. Clin. Invest. 94:1440-1448 (1994). Recombinant AAV vectors have been used successfully for *in vitro* and *in vivo* transduction of marker genes and genes involved in human diseases (*see, e.g.,* Walsh et al,, J. Clin. Invest. 94:1440-1448 (1994)). AAV has a broad host range for infectivity. Details concerning the generation and use of rAAV vectors are described in U.S. Pat. Nos. 5,139,941 and 4,797,368.

Typically, recombinant AAV (rAAV) is made by cotransfecting a plasmid containing the gene of interest flanked by the two AAV terminal repeats and/or an expression plasmid containing the wild-type AAV coding sequences without the terminal repeats, for example pIM45. The cells are also infected and/or transfected with adenovirus and/or plasmids carrying the adenovirus genes required for AAV helper function. rAAV virus stocks made in such fashion are contaminated with adenovirus which must be physically separated from the rAAV particles (for example, by cesium chloride density centrifugation or column chromatography). Alternatively, adenovirus vectors containing the AAV coding regions and/or cell lines containing the AAV coding regions and/or some or all of the adenovirus helper genes could be used. Cell lines carrying the rAAV DNA as an integrated provirus can also be used.

Multiple serotypes of AAV exist in nature, with at least twelve serotypes (AAV1-AAV12) currently known. Despite the high degree of homology, the different serotypes have tropisms for different tissues. The receptor for AAV1 is unknown; however, AAV1 is known to transduce skeletal and cardiac muscle more efficiently than AAV2. Since in most of the studies have been done with pseudotyped vectors in which the vector DNA flanked with AAV2 ITR is packaged into capsids of alternate serotypes, it is clear that the biological differences are related to the capsid rather than to the genomes. Recent evidence indicates that DNA expression cassettes packaged in AAV1 capsids are at least 1 log₁₀ more efficient at transducing cardiomyocytes than those packaged in AAV2 capsids. In some embodiments, the AAVs that do not produce an immune response in the subject are used. In some embodiments, the AAV in the invention is adeno-associated viral vectors 9 (AAV9). As used herein, AAV9.cMLCK vector refers to a cMLCK gene transfer vector based on the recombinant AAV9.

The dosage of the cMLCK-encoding polynucleotide vector in the disclosed methods that will be effective in the treatment of advanced heart failure can be determined by standard clinical techniques. The dosage of the cMLCK-encoding polynucleotide vector in the disclosed methods for expressing the cMLCK protein can also be determined by the clinical effects of the methods. In addition, *in vitro* assays may optionally be employed to help identify optimal dosage ranges. The precise dose to be employed in the formulation will also depend on the route of administration, and the seriousness of the heart failure, and should be decided according to the judgment of the practitioner and each patient's circumstances. In some embodiments, the dose in the disclosed methods is about 1 x 10¹⁰-1 x 10¹⁵gc/patient. In some embodiments, the dose is about 1 x 10¹⁰-5 x 10¹⁴gc/patient. In some embodiments, the dose is about 1 x 10¹¹-1 x 10¹⁴gc/patient. In some embodiments, the dose is about 1 x 10¹¹-5 x 10¹³gc/patient. In some embodiments, the dose is about 5 x 10¹⁰-5 x 10¹¹ gc/patient. In some embodiment, the dose is about 2 x 10¹¹-5 x 10¹¹ gc/patient. In one embodiment, the dose is about 1 x 10¹⁰ gc/patient. In another embodiment, the dose is about 2 x 10¹⁰ gc/patient. In another embodiment, the dose is about 5 x 10¹⁰ gc/patient. In one embodiment, the dose is about 1 x 10¹¹ gc/patient. In another embodiment, the dose is about 2 x 10¹¹ gc/patient. In another embodiment, the dose is about 5 x 10¹¹ gc/patient. In one embodiment, the dose is about 1 x 10¹² gc/patient. In another embodiment, the dose is about 2 x 10¹² gc/patient. In another embodiment, the dose is about 5 x 10¹² gc/patient. In one embodiment, the dose is about 1 x 10¹³ gc/patient. In another embodiment, the dose is about 2 x 10¹³ gc/patient. In another embodiment, the dose is about 5 x 10¹³ gc/patient. In one embodiment, the dose is about 1 x 10¹⁴ gc/patient. In another embodiment, the dose is about 2 x 10¹⁴ gc/patient. In yet another embodiment, the dose is about 5 x 10¹⁴ gc/patient.

In another aspect, the present disclosure provides compositions for cMLCK gene therapy. In some embodiments, the composition is a polynucleotide vector that encodes a cMLCK protein. In some embodiments, the polynucleotide vector is a viral vector. In one embodiment, the viral vector is an adeno-associated viral vectors (AAV). In a specific embodiment, the polynucleotide vector is an AAV9.

In another aspect, the present disclosure provides pharmaceutical compositions. Such compositions comprise a therapeutically effective amount of a cMLCK-encoding polynucleotide vector and a pharmaceutically acceptable carrier. In some embodiments, the polynucleotide vector is a viral vector. In one embodiment, the viral vector is an adeno-associated viral vectors (AAV). In a specific embodiment, the polynucleotide vector is an AAV9. In some embodiments, water is the pharmaceutically acceptable carrier. In some embodiments, saline solutions and aqueous dextrose and glycerol solutions are employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, sustained-release formulations and the like. Examples of suitable pharmaceutical carriers are described in *Remington's Pharmaceutical Sciences* by E.W. Martin. Such compositions will contain a therapeutically effective amount of the therapeutic, preferably in purified form, together with a suitable amount of carrier so as to provide the form for proper administration to the patient. The formulation should suit the mode of administration.

In a specific embodiment, the composition is formulated in accordance with routine procedures as a pharmaceutical composition adapted for intravenous administration or intracoronary infusion to human beings. Typically, compositions for intravenous administration or intracoronary infusion are solutions in sterile isotonic aqueous buffer. Generally, the ingredients are supplied either separately or mixed together in unit dosage form, for example, as a dry lyophilized powder or water free concentrate in a hermetically sealed container such as an ampoule or sachet indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

In another aspect, the present disclosure provides compositions disclosed herein for use in the various disclosed methods. In some embodiments, the compositions disclosed herein are used for treating advanced heart failure in a subject, comprising administering to the subject an effective amount of a polynucleotide vector that encodes a cMLCK protein, expressing the cMLCK protein from the polynucleotide vector in the heart cells of the subject, and improving ventricular function of the heart. In some embodiments, the composition is a polynucleotide vector that encodes a cMLCK protein. In some embodiments, the polynucleotide vector is a viral vector. In one embodiment, the viral vector is an adeno-associated viral vectors (AAV). In a specific embodiment, the polynucleotide vector is an AAV9.

In other embodiments, the compositions disclosed herein are used for expressing a cMLCK protein in a subject, comprising administering to the subject a polynucleotide vector that encodes the cMLCK protein and expressing the cMLCK protein from the polynucleotide vector in the heart cells of the subject, wherein the subject has advanced heart failure. In some embodiments, the composition is a polynucleotide vector that encodes a cMLCK protein. In some embodiments, the polynucleotide vector is a viral vector. In one embodiment, the viral vector is an adeno-associated viral vectors (AAV). In a specific embodiment, the polynucleotide vector is an AAV9.

The invention is illustrated by the following examples which are not intended to be limiting in any way.

### EXAMPLE 1

### AAV9.cMLCK Viral Vector Construction, Production, and Quality Control

This example describes the construction, production, and quality control of the cMLCK-encloding polynucleotide in an AAV9 vector.

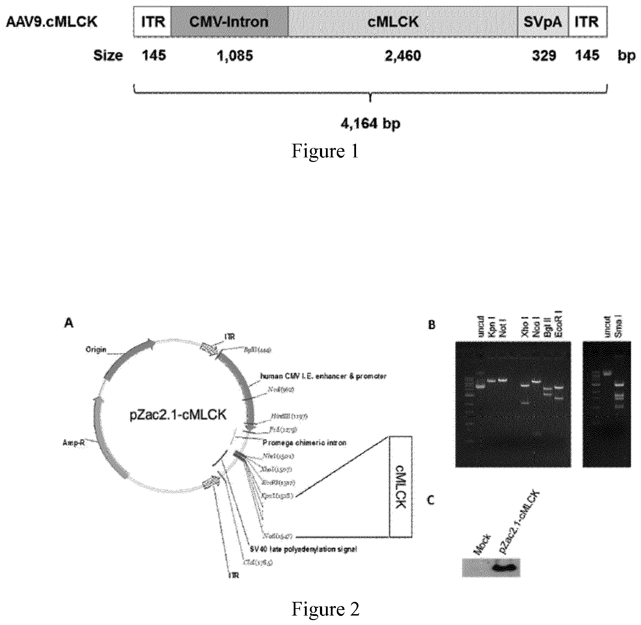

The *cis* plasmid pZac2.1-cMCLK, in which human cMLCK (hcMLCK)-encoding polynucleotide (SEQ ID NO:4) transgene expression was driven by human cytomegalovirus (CMV) promoter, was constructed. Figure 1 shows the genome structure of the cMLCK-encoding polynucleotide in an AAV9 vector. hcDNA for cMLCK (SEQ ID No:4) was cloned into the Kpn I/Not I site of AAV9 cis plasmid pZac2.1-CMV. Expression of cMLCK was under the control of the CMV immediate early promoter/enhancer and the poly adenylation site of SV40.

293 cells were transfected with *cis* plasmid pZac2.1-hcMCLK, *trans* plasmid pAAV2/9 containing the AAV2 rep gene and capsid protein genes from AAV9, and adenovirus helper plasmid pAdAF6. The cells were purified by gradient ultracentrifugation with increasing iodixanol concentration of 15%, 25%, 40%, and 54%, and purity analysis was conducted by SDS-PAGE and endotoxin assay (release criteria is <5 endotoxin units/ml for large animal studies). Figure 2 shows the construction and quality control of *cis* plasmid pZac2.1-cMLCK. Plasmid DNA purity and concentration were determined by absorbance at 260 nm and 280 nm, and identity of cMLCK cDNA and regulatory elements (ITR, CMV enhancer and promoter, intron, and SV40 polyA) was confirmed by restrictive enzyme digestion and full-length sequencing in both strands. Human cMLCK transgene expression was further confirmed by western blotting after transfecting human embryonic kidney 293 cells (HEK293 cells) with pZac2.1-cMLCK.

The AAV9.cMLCK was obtained successfully. The data of one batch were listed as follows. *Lot* #*1:* Genome titer: 1.68 x 10¹³ GC/ml; Yield: 3.19 x 10¹³ GC; Purity: 100%; Endotoxin: <1 EU/ml.

### EXAMPLE 2

### cMLCK Gene Expression in Vitro

Example 2 describes the expression of cMLCK transgene *in vitro,* including in HEK293 cells, H9C2 cells (a cardiac cell line), and cardiac myocytes from neonatal rats.

The expression of cMLCK transgene at mRNA and/or protein levels were confirmed in HEK293 cells and H9C2 cells. Quantitative RT-PCR was performed to measure mRNA level of cMLCK, and western blotting was performed using cells lysates to measure protein contents of cMLCK.

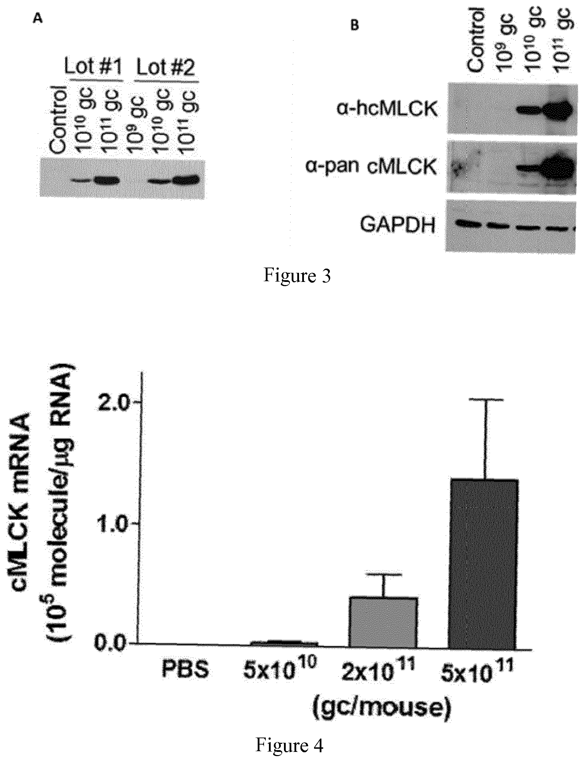

After confirming cMLCK transgene expression in HEK293 cells and H9C2 cells, the cMLCK transgene expression in isolated cardiac myoyctes was further evaluated. Cardiac myocytes were isolated from neonatal rats as previously described in Lai et al., Hum Gene Ther, 23(3):255-261 (2012). Total RNA was isolated from cells using STAT-60 (Tel-Test, TX), followed by RNeasy Mini kit (Qiagen) purification and DNase treatment to eliminate residual genomic DNA contamination. Quantitative RT-PCR was performed to compare mRNA content of cMLCK. Western blotting was performed using cells lysates to measure protein contents of cMLCK transgene expression. Figure 3 shows the cMLCK transgene was expressed in both HEK293 cells (Figure 3A) and isolated cardiac myocytes (Figure 3B, Lot #1 AAV9.cMLCK) after AAV9.cMLCK gene transfer. Different dosages of AAV9.cMLCK were used to infect cells and cardiac myocytes, GAPDH was used as an internal control.

### EXAMPLE 3

### Optimization of Conditions for Cardiac Gene Transfer of AAV9.cMLCK

Example 3 describes a series of studies to optimize the conditions for AAV9.cMLCK gene transfer.

Indirect coronary injection was used to deliver AAV9.cMLCK into mice. Male C57BL/6J mice (The Jackson Laboratories, Bar Harbor, ME) aged 10-12 weeks were anesthetized and intubated. A thoracotomy was performed between the second and third rib space and the proximal aorta and pulmonary artery were isolated and occluded using a vascular clamp (Roth et al., Am J Physiol Heart Circ Physiol, 287(1):H172-177 (2004)). AAV9.cMLCK was injected into the LV cavity. The clamp was released 60 sec after occlusion.

The biodistribution of viral vector and cMLCK transgene were accessed by PCR and RT-PCR. Genomic DNA was isolated from a variety of organs (LV, lung, liver, skeletal muscle, spleen, intestine, kidney, stomach, and gonads) using DNeasy Mini kit (Qiagen).

Quantitative PCR was performed to quantitate DNA copies of AAV9.cMLCK in each organ. Total RNA was isolated from a variety of organs using STAT-60 (Tel-Test, TX), followed by RNeasy Mini kit (Qiagen) purification and DNase treatment to eliminate residual genomic DNA contamination. Quantitative RT-PCR was performed to compare mRNA content of cMLCK. Western blotting was performed using LV homogenates to measure protein contents of cMLCK transgene expression.

The mRNA and protein expression of cMLCK transgene in the heart 2, 5, 10 weeks was compared after injection of 0, 5x10¹⁰, 2x10¹¹, and 5x10¹¹ gc/mouse AAV9.cMLCK. Figure 4 shows the quantification of cMLCK transgene mRNA expression in LV samples 5 weeks after indirect coronary gene transfer of AAV9.cMLCK. There were 1.41 x 10⁵ cMLCK transgene mRNA molecules per µg total RNA 5 weeks after indirect gene transfer of 5 x 10¹¹ gc/mouse AAV9.cMLCK.

The dose-dependent cMLCK transgene expression in LV samples was found after coronary gene delivery. The results showed decent cMLCK transgene expression occurred after injection of 5x10¹¹ gc/mouse AAV9.cMLCK.

### EXAMPLE 4

### AAV9.cMLCK Gene Transfer in Improving Cardiac Function in Pressure-overloaded Mouse Hearts

Example 4 describes the effect of AAV9.cMLCK gene transfer in improving cardiac function in pressure-overloaded mouse hearts. Chronic pressure overload, such as occurs with persistent hypertension, is associated with a higher risk of the development of clinical heart failure. Although this process generally takes decades to develop in patients, severe LV pressure overload associated with transverse aortic constriction (TAC) leads to LV hypertrophy and impaired LV function in weeks in mice. Pressure overload provides an efficient model to study the effects of AAV9.cMLCK gene transfer on the pressure-stressed heart.

Transverse aortic constriction (TAC) was performed on a cohort of C57BL/6N mice. Mice were anesthetized with 5% isoflurane in oxygen (1 l/min), intubated, and ventilated (pressure-controlled). Anesthesia was maintained with 1% isoflurane in oxygen. The chest was entered at the second intercostal space at the left upper sternal border, and a segment of the aortic arch between the innominate and left carotid arteries was dissected. A 7-0 silk suture was tied against a 27-gauge needle, which yield a substantial aortic constriction.

Three weeks after TAC, echocardiography was performed on the surviving mice to assess chamber dimensions and LV function. These mice were randomly divided into two groups. One group of mice received AAV9.cMLCK gene transfer by indirect intracoronary delivery, and another group of mice received PBS injection by the same way. Five weeks after gene transfer, chamber dimensions and LV function were assessed again by echocardiography. A series of echocardiography were performed in anesthetized mice. LV chamber dimensions, LVEF, and velocity of circumferential fiber shortening (Vcf) were determined. In addition, LV contractile function and relaxation were determined by *in vivo* hemodynamics. A 1.4F micromanometer catheter (Millar Instruments) was inserted *via* the right carotid artery and advanced into the LV of the anesthetized animals. LV pressure, LV ±dP/dt, end-systolic pressure-volume relationship (ESPVR), stroke volume, cardiac output, and systemic vascular resistance were determined.

Body weight, LV weight, lung weight, liver weight, and tibial length were determined at necropsy. Diastole arrested LV samples were divided into 3 pieces: a short-axis midwall LV ring was fixed in formalin for determination of cardiac myocyte size, cardiac apoptosis, and fibrosis, and the other 2 pieces were quickly frozen in liquid nitrogen and stored at -80°C for biochemistry and molecular biology studies.

LV sections were stained with hematoxylin & eosin. Random fields from both the septum and LV free wall were photographed by an observer blinded to the group identity. Cardiac myocyte cross-sectional area was determined using NIH Image J software.

Terminal deoxynucleotidyl transferase-mediated dUTP nick end-labeling (TUNEL) assay was performed on LV sections using the CardioTACS In Situ Apoptosis Detection Kit (R&D Systems). DNA fragmentation assay was used to confirm changes in apoptosis rate. Activity of caspase 3/7 was measured using Caspase-Glo 3/7 Assay kit (Promega).

LV sections were stained with picrosirius red. Collagen deposition area was quantified using NIH Image J software and fractional collagen area was calculated. Expression of collagen I, collagen III, periostin, MMPs (matrix metalloproteinases), and TIMPs (tissue inhibitors of matrix metalloproteinases) were measured by quantitative RT-PCR and Western blotting.

Total RNA was isolated from LV samples using STAT-60 (Tel-Test, TX), followed by RNeasy Mini kit (Qiagen) purification and DNase treatment to eliminate residual genomic DNA contamination. Quantitative RT-PCR was performed to compare mRNA content of regulators of Ca²⁺ handling (PLN, SERCA2a, calsequestrin, RyR2, FKBP12, L-type Ca²⁺ channel, and NCX), contractility (cTnI, cTnC, cTnT, and tropomysin), hypertrophy (ANF, BNP, α-SK actin, β-MHC, FHL1, MEF2D), apoptosis (Pim1, sFRP1, Bcl2, Bax, and caspase 3), and fibrosis (collagen I, collage III, periostin, MMPs, and TIMPs).

Western blotting was performed using LV homogenates to measure protein contents of regulators of Ca²⁺ handling (PLN, phospho-PLN, SERCA2a, calsequestrin, RyR2, phosphor-RyR2, FKBP12, L-type Ca²⁺ channel, and NCX), contractility (cTnI, phospho-cTnI, cTnC, cTnT, and tropomysin), hypertrophy (FHL1, MEF2D), apoptosis (Pim1, sFRP1, Wif1, Bcl2, Bax, and caspase 3), and fibrosis (collagen and periostin). Immunofluorescence microscopy was performed to determine expression of FHL1, MEF2D, PLN, Pim1, sFRP1, and WIF1.

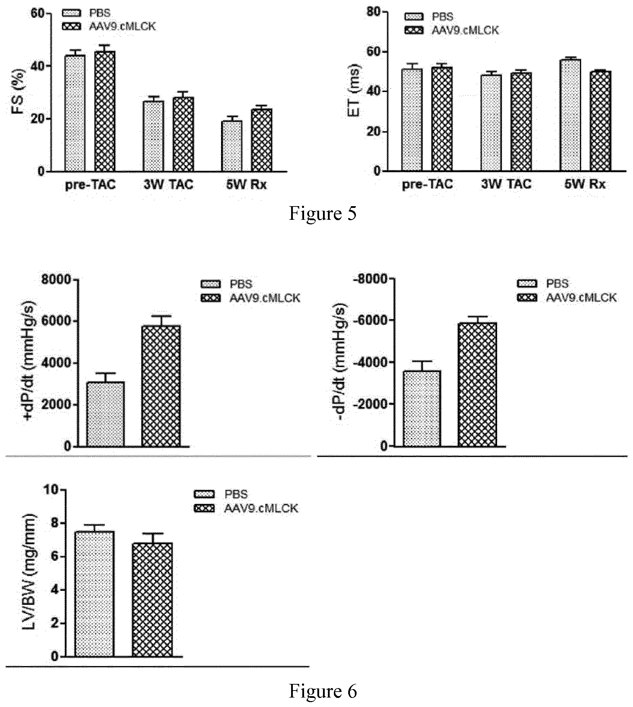

Figure 5 shows the echocardiography detection before and after AAV9.cMLCK gene transfer. As shown as Figure 5, the average FS for the group with AAV9.cMLCK gene transfer significantly increased and ET decreased comparing with those for the group with PBS injection, suggesting that AAV9.cMLCK gene transfer could improve LV systolic function of the mice suffering clinical heart failure resulted from TAC.

Figure 6 shows the *in vivo* hemodynamics determination after AAV9.cMLCK gene transfer.

As shown as Figure 6, the average +dP/dt for the group with AAV9.cMLCK gene transfer significantly increased comparing with them for the group with PBS injection, suggesting that AAV9.cMLCK gene transfer could improve LV systolic function of the mice suffering clinical heart failure resulted from TAC. The significant increasing of the average -dP/dt indicated that AAV9.cMLCK gene transfer could improve LV diastolic function of TAC model. The average LV/BW decreased after AAV9.cMLCK gene transfer, suggesting that AAV9.cMLCK gene transfer could partly improve myocardial hypertrophy resulted from clinical heart failure.

### EXAMPLE 5

### Treatment of Advanced Heart Failure Patients Using cMLCK Gene Transfer

Example 5 describes treatment of advanced heart failure patients using gene transfer of AAV9.cMLCK.

Patients with NYHA Class III or IV heart failure or a plasma level of N-terminal pro-brain natriuretic peptide (NT-proBNP) of more than 4000 fmol/ml, or patients that are not responsive to neuregulin treatment, are treated with gene transfer of AAV9.cMLCK.

AAV9.cMLCK are locally administered to the heart of patients via intracoronary infusion at doses of 1 x 10¹¹gc/patient to 5 x 10¹³gc/patient. In general, percutaneous intracoronary delivery is accomplished using standard catheters (5 Fr or 6 Fr guide or diagnostic) and infused using the MEDRAD Mark V ProVis angiographic injection system (Indianola, PA). Infusions occur over a 10-minute period in a cardiac catheterization laboratory after angiography. Standard catheter engagement technique with the coronary arteries is accomplished in the usual fashion with angiographic confirmation of good coaxial position and secure intubation to assure forward infusion antegrade into the coronary circulation.

After 180 days, the ventricular function of these patients are monitored, such as EF values of the left ventricle, Left Ventricular End-Diastolic Volume (LVEDV), or Left Ventricular End-Systolic Volume (LVESV) of the patients, by echocardiography. The patients are assessed for improvements of ventricular function of the heart using such parameters.

All publications, patents and patent applications cited in this specification are herein incorporated by reference as if each individual publication or patent application were specifically and individually indicated to be incorporated by reference. Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

## Claims

1. A method for treating advanced heart failure in a human subject, comprising administering to the subject an effective amount of a polynucleotide vector that encodes a cMLCK protein, expressing the cMLCK protein from the polynucleotide vector in the heart cells of the subject, and improving ventricular function of the heart.

2. The method of claim 1, wherein the subject has a NYHA Class III heart failure.

3. The method of claim 1, wherein the subject has a NYHA Class IV heart failure.

4. The method of claim 1, wherein the subject has a plasma level of N-terminal pro-brain natriuretic peptide (NT-proBNP) of more than 4000 fmol/ml.

5. The method of claim 1, wherein the subject is not responsive to neuregulin treatment.

6. The method of claim 1, wherein the cMLCK protein has an amino acid sequence comprising SEQ ID NO:1.

7. The method of claim 1, wherein the polynucleotide vector is locally administered to the subject.

8. The method of claim 1, wherein the AAV is adeno-associated viral vectors 9 (AAV9).

9. The method of claim 1, wherein the EF value of the left ventricle of the subject is enhanced by at least 5%.

10. The method of claim 1, wherein the Left Ventricular End-Diastolic Volume (LVEDV) or Left Ventricular End-Systolic Volume (LVESV) of the subject is reduced by at least 5%.
